# EUROPEAN PATENT APPLICATION

(11) **EP 4 140 293 A1**
(43) Date of publication of application: **01.03.2023**
(21) Application number: 21791966.1
(22) Date of filing: 21.04.2021
(51) Int. Cl.: A01H 5/00, A01H 6/82, A24B 13/00, A24B 15/00, C12N 15/10, C12N 15/113, C12N 15/29

(54) **TOBACCO PLANT BODY AND METHOD FOR PRODUCING SAME**

(30) Priority: 21.04.2020 JP 2020075565
(71) Applicant: Japan Tobacco Inc., Tokyo 105-6927 (JP)
(72) Inventor: MAGOME, Hiroshi, Tokyo 130-8603 (JP); ARAI, Masao, Tokyo 130-8603 (JP); OYAMA, Kiyoshi, Tokyo 130-8603 (JP); TAKAKURA, Yoshimitsu, Tokyo 130-8603 (JP); NISHIGUCHI, Ryo, Tokyo 130-8603 (JP)
(74) Representative: Hoffmann Eitle
(86) International application number: PCT/JP2021/016145
(87) International publication number: WO 2021/215465

(57) **Abstract**

The object of an embodiment of the present invention is to improve the quality of a tobacco product. The present invention relates to a tobacco plant in which a mutation is introduced in a genome, the mutation causing suppression of a function of a specific endogenous gene.

## Description

### Technical Field

The present invention relates to (i) a tobacco plant which allows an improvement in the quality of a tobacco leaf, (ii) a method of producing the tobacco plant, (iii) a harvest from the tobacco plant, and (iv) a processed product of the harvest.

### Background Art

Carotenoids collectively refer to red, yellow, or, orange biopigments, and examples of plant carotenoids include β-carotene and lutein. Breakdown products resulting from degradation of carotenoids are collectively referred to as apocarotenoids. The apocarotenoids, which are products resulting from the degradation of the carotenoids, includes aroma components (such as β-damascenone, β-ionone, and megastigmatrienone).

Carotenoids are known as substances (antioxidants and precursors of vitamin A, as nutrients) which are useful in keeping biological functions normal. Therefore, various crops in which increased amounts of carotenoids are accumulated by genetic modifications (GM) are produced for commercial use and for improvement in stress tolerance of plants.

As examples of a method of increasing the amount of a carotenoid accumulated in a tobacco plant, known are (i) administering a chemical agent to the plant and (ii) overexpressing, by a genetic modification, a gene which is involved in biosynthesis or metabolism of the carotenoid (Patent Literature 1). However, administration of a chemical agent has a problem in elimination of the chemical agent from a plant and a product. Further, in a case where an exogenous gene is introduced into a plant by a genetic modification, it is not possible to control a position in a genome at which position the exogenous gene is inserted.

Carotenoid cleavage dioxygenases (CCDs) are oxygenases which cleave carotenoids serving as substrates so that apocarotenoids are produced. It is known, based on the sequence homologies of the apocarotenoids, that nine kinds (some of which are designated "NCED" according to numbering) of CCD genes are widely conserved in higher plants. Of these genes, CCD1 and CCD4 have been reported to be involved in, in a plurality of plant species, regulation of carotenoid contents and/or production of apocarotenoids.

For example, with regard to solanaceous plants (including tobacco), there are reports on petunia CCD1, tomato CCD1, and potato CCD4. With regard to petunia and tomato, suppression of expression of CCD1 genes has been reported to cause β-ionone to be decreased in flowers. With regard to potato, suppression of functions of CCD4 genes has been reported to cause carotenoids to be accumulated in flowers and tubers. Apart from solanaceous plants, suppression of expression of CCD4 genes has been reported to cause carotenoids to be accumulated in parts of plants.

With regard to tobacco, there is a report that a CCD gene family in tobacco has been examined by a comprehensive analysis of whole genomic information with use of homology searches (Non-Patent Literature 1). In Non-Patent Literature 1, the presence of three CCD4 genes in tobacco and expression of these three CCD4 genes in tobacco plants have been reported.

### Citation List

### [Patent Literature]

[Patent Literature 1]
Specification of U.S. Patent Application Publication, No. 2014/0289901

### [Non-patent Literature]

[Non-patent Literature 1]
Zhou et al (2019) Carotenoid Cleavage Dioxygenases: Identification, Expression, and Evolutionary Analysis of This Gene Family in Tobacco, International Journal of Molecular Sciences, vol. 20, no. 22. Article number 5796

### Summary of Invention

### Technical Problem

The aroma and taste of a tobacco product is a complex quality which appeals to human's sense organs, and is considered to be based on the balance of various components contained in a tobacco leaf as a material. Since a plurality of genes having similar functions are present in a plant, it is very difficult to regulate various components as intended.

The object of an aspect of the present invention is to realize a tobacco plant which allows an improvement in the quality of a tobacco product.

### Solution to Problem

In view of the above object, the inventors of the present invention analyzed, in detail, components contained in a tobacco plant in which a function of a specific endogenous gene had been suppressed, and actually examined the quality of a tobacco product. As a result, the inventors completed the present invention.

In order to attain the above object, a tobacco plant in accordance with an aspect of the present invention is a tobacco plant in which a mutation is introduced in a genome, the mutation causing suppression of a function of at least any one of: an endogenous gene which contains, as a coding region, a polynucleotide that encodes a polypeptide having a sequence identity of 80% or higher with an amino acid sequence represented by SEQ ID NO: 1; an endogenous gene which contains, as a coding region, a polynucleotide that encodes a polypeptide having a sequence identity of 80% or higher with an amino acid sequence represented by SEQ ID NO: 2; and an endogenous gene which contains, as a coding region, a polynucleotide that encodes a polypeptide having a sequence identity of 80% or higher with an amino acid sequence represented by SEQ ID NO: 3.

Further, a method of producing a tobacco plant in accordance with an aspect of the present invention is a method of producing a tobacco plant, including the step of introducing a mutation into a genome of a tobacco plant, the mutation causing suppression of a function of at least any one of: an endogenous gene which contains, as a coding region, a polynucleotide that encodes a polypeptide having a sequence identity of 80% or higher with an amino acid sequence represented by SEQ ID NO: 1; an endogenous gene which contains, as a coding region, a polynucleotide that encodes a polypeptide having a sequence identity of 80% or higher with an amino acid sequence represented by SEQ ID NO: 2; and an endogenous gene which contains, as a coding region, a polynucleotide that encodes a polypeptide having a sequence identity of 80% or higher with an amino acid sequence represented by SEQ ID NO: 3.

### Advantageous Effects of Invention

According to an aspect of the present invention, it is possible to improve the quality of a tobacco leaf harvested from a tobacco plant.

### Brief Description of Drawings

Fig. 1 is a graph showing the results of comparing the contents of carotenoids in plants prepared in Examples.
Fig. 2 is a graph showing the results of comparing the contents of carotenoids in *Nicotiana sylvestris* plants prepared in Examples.
Fig. 3 is a graph showing the results of comparing the contents of carotenoids in cured leaves of the plants prepared in Examples.
Fig. 4 is a graph showing the results of comparing the contents of carotenoids in the other mutant plants grown in a greenhouse.
Fig. 5 is a graph showing the results of comparing the contents of carotenoids in cured leaves of the other mutant plants grown on a field.

### Description of Embodiments

### [1. Tobacco plant]

An embodiment of the present invention provides a tobacco plant in which a mutation is introduced in a genome, the mutation causing suppression of a function of at least any one of: an endogenous gene which contains, as a coding region, a polynucleotide that encodes a polypeptide having a sequence identity of 80% or higher with an amino acid sequence represented by SEQ ID NO: 1; an endogenous gene which contains, as a coding region, a polynucleotide that encodes a polypeptide having a sequence identity of 80% or higher with an amino acid sequence represented by SEQ ID NO: 2; and an endogenous gene which contains, as a coding region, a polynucleotide that encodes a polypeptide having a sequence identity of 80% or higher with an amino acid sequence represented by SEQ ID NO: 3.

The tobacco plant shows a change (including an increase and a decrease) in the content of at least one of carotenoids and apocarotenoids, as compared with a wild-type plant. The tobacco plant shows, as indicated in Examples described later, a change (including an increase and a decrease) in the content of at least one of carotenoids and apocarotenoids in its leaves (fresh leaves and cured leaves), as compared with a wild-type plant. Therefore, the balance of various components contained in the tobacco plant differs from that in a conventional tobacco plant, and such a balance causes an improvement in the aroma and taste (quality) of a tobacco product obtained from the tobacco plant. Note, here, that the tobacco product the aroma and taste (quality) of which is improved may be any of cigarettes, cigars, heated tobaccos (non-burning type flavor inhalers), and smokeless tobaccos. Note also that a processed product of a tobacco leaf obtained from the tobacco plant can be also used as a flavor source for electronic cigarettes and the like.

The content of each of the carotenoids and the apocarotenoids in the tobacco plant may be increased or decreased, provided that the aroma and taste (quality) of the tobacco product produced from the tobacco plant is improved. In a preferred embodiment of the tobacco plant which makes it possible to produce a tobacco product having improved aroma and taste, the tobacco plant has an increased total carotenoid content, as compared with a wild-type tobacco plant. The term "total carotenoid content" as used herein refers to the contents of all the carotenoids in the tobacco plant which contents are quantified by absorptiometry. The total carotenoid content of the tobacco plant is, for example, 1.2 times or more, 1.5 times or more, 1.7 times or more, 1.8 times or more, 1.9 times or more, preferably 2.0 times or more, 2.5 times or more, and more preferably 3 times or more as high as the total carotenoid content of a wild-type tobacco plant.

In the above preferred embodiment, the tobacco plant more preferably has an increased content of at least one of lutein, β-carotene, and zeaxanthin, as compared with a wild-type tobacco plant. Each of these carotenoid contents of the tobacco plant is, for example, 1.2 times or more, 1.5 times or more, 1.7 times or more, 1.8 times or more, 1.9 times or more, preferably 2.0 times or more, 2.5 times or more, and more preferably 3 times or more as high as a corresponding one of the carotenoid contents of a wild-type tobacco plant. These carotenoid contents can be quantified by a publicly known technique such as high-performance liquid chromatography.

In the above preferred embodiment, the tobacco plant more preferably has an increased content of at least one of β-ionone and dihydroactinidiolide (each of which is an apocarotenoid), as compared with a wild-type tobacco plant. These apocarotenoids are known as floral aroma components.

Apocarotenoids which are partially allowed to be decreased in the contents can be, for example, apocarotenoids which are known as aroma components (such as β-damascenone, megastigmatrienone (and structural isomers thereof), and 3-hydroxy-β-damascone). Among them, the apocarotenoids can be β-damascenone, megastigmatrienone (and structural isomers thereof), and 3-hydroxy-β-damascone.

A tobacco leaf obtained from the tobacco plant or a processed product of the tobacco leaf can exhibit, in accordance with the contents of the carotenoids, a color (in particular, yellow or orange) different from that of a tobacco leaf obtained from the tobacco plant in which suppression of the function of at least any one of the above endogenous genes does not occur or a processed product of the tobacco leaf. In the case of, in particular, a smokeless tobacco, there are many opportunities at which a tobacco leaf or a processed product of the tobacco leaf is directly viewed by a user. Therefore, the tobacco leaf or the processed product of the tobacco leaf which has a different color can give a user an impression visually different from a conventional impression, without being colored.

In an embodiment, the tobacco plant which has an increased content of lutein, β-carotene, or zeaxanthin as compared with a wild-type plant can be a material of a tobacco leaf and a tobacco product from each of which reduced amounts of TSNAs are produced. Lutein, β-carotene, and zeaxanthin are known as antioxidants. Antioxidants suppress production of tobacco-specific nitrosamines (TSNAs). Each TSNA [N'-nitrosonornicotine (NNN), N'-nitrosoanatabine (NAT), N'-nitrosoanabasine (NAB), 4-(methylnitrosamino)-1-(3-pyridyl)-1-butanone (NNK), or the like] is produced by a nonenzymatic reaction of nornicotine, anatabine, anabasine, or nicotine with nitrous acid or the like (nitrosation). Such a reaction can be inhibited by a carotenoid which is a kind of antioxidant. That is, lutein, β-carotene, or zeaxanthin can reduce the amounts of TSNAs produced from a tobacco leaf and a tobacco product. Note, here, that the tobacco product from which reduced amounts of TSNAs are produced may be any of cigarettes, cigars, heated tobaccos (non-burning type flavor inhalers), and smokeless tobaccos.

As used herein, the "tobacco plant" and the "tobacco" encompass (i) entire individuals (such as a mature plant, a seedling, and a seed), (ii) tissues (such as a leaf, a stem, a flower, a root, a reproductive organ, an embryo, and a part of any of these), and (iii) cured products of any of these.

As used herein, the term "sequence identity (of an amino acid sequence)" means a percentage at which a concerned (amino acid) sequence matches a reference (amino acid) sequence. Note, here, that a part of the sequence which part does not match is a part at which an amino acid residue is substituted, added, deleted, or inserted.

Note, here, that the expression "polypeptide having a sequence identity of 80% or higher with an amino acid sequence represented by [...]", which specifies a polypeptide with use of one of amino acid sequences listed in a sequence listing, means a wild-type polypeptide. The wild-type polypeptide means a polypeptide which is typically present in a *Nicotiana* plant (described later). The wild-type polypeptide is, for example, a protein which has the amino acid sequence represented by SEQ ID NO: 1, 2, or 3 or an orthologue, in the *Nicotiana* plant, of the protein. As used herein, the terms "polypeptide" and "protein" have substantially the same meaning, and can therefore be used interchangeably.

As used herein, the term "endogenous gene" means a gene which is originally present in a genome of a *Nicotiana* plant. That is, the endogenous genes are each not an exogenous gene which is present in a plant other than a *Nicotiana* plant.

Therefore, the polypeptide which is present in a decreased amount in the tobacco plant need only be a polypeptide having a sequence identity of 80% or higher with each of the amino acid sequences listed in the sequence listing. A higher sequence identity is more preferable (e.g., 81%, 82%, 83%, 84%, 85%, 86%, 87%, 88%, 89%, 90%, 91%, 92%, 93%, 94%, 95%, 96%, 97%, 98%, or 99% or higher).

In a *Nicotiana* plant, genes corresponding to the endogenous genes, the function of at least any one of which is suppressed by the above mutation, have high sequence identities, and are highly conserved. That is, polypeptides respectively encoded by the genes (orthologues, in the *Nicotiana* plant, of the above polypeptides respectively encoded by the endogenous genes) have the above-described sequence identities. Examples of sequence identities of orthologues, in *Nicotiana* plants and the other plants, of the endogenous genes are shown in Table 1. Table 1 shows the sequence identities of the orthologues in these plants with respect to a tobacco CCD4-S protein (SEQ ID NO: 1), a tobacco CCD4-T1 protein (SEQ ID NO: 2), and a tobacco CCD4-T2 protein (SEQ ID NO: 3). In this specification, the "wild-type tobacco plant" may be, but is not limited to, a plant in which a factor that suppresses expression of a tobacco CCD4-S gene, a tobacco CCD4-T1 gene, and a tobacco CCD4-T2 gene is not introduced and which does not have a mutation in any of these genes.

**[Table 1]**

| | *N. sylvestris* | *N. tomentosiformis* | *N. attenuata* | Tomato | Potato | Arabidopsis | Rice |
|---|---|---|---|---|---|---|---|
| Tobacco CCD4-S | 100% | 96% | 96% | 83% | 65% | 65% | 58% |
| Tobacco CCD4-T1 | 96% | 100% | 96% | 82% | 66% | 66% | 57% |
| Tobacco CCD4-T2 | 95% | 100% | 95% | 82% | 65% | 64% | 55% |

The "decreased amount" of the polypeptide means that the polypeptide is present at a percentage of 70% or less, 60% or less, 50% or less, 40% or less, 30% or less, 20% or less, 10% or less, 5% or less, or 1% or less with respect to the amount of a wild-type polypeptide. The amount of the polypeptide with respect to the amount of the wild-type polypeptide can be selected, as appropriate, from the above-described values which cause a change in the content of at least one of the carotenoids and the apocarotenoids in the tobacco plant.

Note that it is preferable that the above-described decrease in the amount of the polypeptide present in the tobacco plant be genetically and stably inherited by a cultured cell, a callus, protoplast, a seed, and offspring each of which is obtained from the tobacco plant. Therefore, the tobacco plant can be an individual developed from the cultured cell, the callus, the protoplast, the seed, or the offspring, each of which has been produced through an artificial operation. Thus, these materials, from each of which the individual is developed, are also encompassed in the scope of the present invention.

The tobacco plant can further encompass bred progeny obtained by crossing. Breeding with use of mutants has been performed in many plant species, including rice, wheat, barley, and soybean. For example, a mutant isolated from a mutant population which has been obtained by a treatment with use of a mutagen has multiple mutations in a region other than a region of a target gene. In general, therefore, backcrossing is performed to remove an excess mutation(s). In this crossing, a desired character (a change in the content of at least one of the carotenoids and the apocarotenoids) of the mutant can be introduced into an existing cultivar by crossing the mutant with the cultivar having an excellent character. Bred progeny thus obtained can be a variety obtained by adding high value to an existing cultivar.

Note, here, that the desired character of the mutant is derived from mutations introduced into a plurality of positions (e.g., a plurality of genes) in a genome. For efficient backcrossing, it is therefore necessary to select, in advance, an individual having the mutations. In the selection of the individual, it is advantageous to be able to easily detect (i) whether or not the mutations are present in the individual and (ii) whether the mutations are homozygous or heterozygous. The mutations can be detected by a method (described later) for detecting a mutation in a gene. Apart from the perspective above, it is preferable that a line having a high cultivar-return-rate (i.e., the proportion of a cultivar-derived genomic region to an entire genomic region) be obtained with the fewer times of crossing. The fewer times of crossing can be achieved by, for example, marker assisted selection (MAS) in which a background marker indicative of a polymorphism between the mutant and the existing cultivar is used. The background marker indicative of the polymorphism can be, for example, an SNP marker or a simple sequence repeat (SSR) marker each of which is known in tobacco. Other than these existing markers, a new marker can be used which encompasses the following differences (a) and (b) each of which is identified by determining respective genome sequences of the mutant and the existing cultivar used in crossing and then making a comparison between the genome sequences: (a) a difference in nucleotide sequence and (b) a difference in the number of repeat sequences in a genome.

The endogenous genes and the genome will be described below by taking *Nicotiana tabacum* (*N. tabacum*) as a reference. *Nicotiana tabacum* (*N. tabacum*), which serves as a reference in the description below, is an amphidiploid, and has both an S genome and a T genome derived from *Nicotiana sylvestris* and *Nicotiana tomentosiformis*, respectively, each of which is an ancestor species thereof. It is known that one of the above three endogenous genes, the function of at least any one of which is suppressed, is present in the S genome (hereinafter, the one will be referred to as a CCD4-S gene) and the other two of the above three endogenous genes are present in the T genome (hereinafter, the two will be respectively referred to as a CCD4-T1 gene and a CCD4-T2 gene).

Note that, with regard to tobacco plants, nucleotide sequences, in coding regions, of part (not the whole) of genes encoding polypeptides which have the substantially same function among species can have (i) 1% to several % difference therebetween when cultivars are compared and (ii) approximately 10% or lower difference therebetween when a cultivar and a wild relative are compared.

A polypeptide having the amino acid sequence represented by SEQ ID NO: 1 is encoded by, for example, a polynucleotide having a nucleotide sequence represented by SEQ ID NO: 4 (by a coding region (CDS) of the CCD4-S gene). A polypeptide having the amino acid sequence represented by SEQ ID NO: 2 is encoded by, for example, a polynucleotide having a nucleotide sequence represented by SEQ ID NO: 5 (by a coding region (CDS) of the CCD4-T1 gene). A polypeptide having the amino acid sequence represented by SEQ ID NO: 3 is encoded by, for example, a polynucleotide having a nucleotide sequence represented by SEQ ID NO: 6 (by a coding region (CDS) of the CCD4-T2 gene).

Note, here, that examples of a method which is for isolating an orthologous gene and which is well known to a person skilled in the art encompass a hybridization technique (Southern, E. M., Journal of Molecular Biology, Vol. 98, 503, 1975) and a polymerase chain reaction (PCR) technique (Saiki, R. K., et al. Science, vol. 230, 1350-1354, 1985, Saiki, R. K. et al. Science, vol. 239, 487-491, 1988). Therefore, a person skilled in the art can easily isolate an orthologous gene of the CCD4-S gene from various plants while, for example, (i) by using, as a probe, the polynucleotide having the nucleotide sequence represented by SEQ ID NO: 4 or a part of the polynucleotide or (ii) by using, as a primer, an oligonucleotide which hybridizes with the polynucleotide under stringent conditions. A person skilled in the art who has read the above description can easily isolate an orthologous gene of the CCD4-T1 gene on the basis of the nucleotide sequence represented by SEQ ID NO: 5 (or a part of the nucleotide sequence), and can easily isolate an orthologous gene of the CCD4-T2 gene on the basis of the nucleotide sequence represented by SEQ ID NO: 6 (or a part of the nucleotide sequence).

Note, here, that the stringent conditions indicate, in general, conditions under which (i) a double-stranded polynucleotide which is specific to a nucleotide sequence is formed but (ii) formation of a double-stranded polynucleotide which is not specific to the nucleotide sequence is markedly suppressed. In other words, the stringent conditions can be such that hybridization is carried out at a temperature falling within a range from (i) a melting temperature (Tm) of a hybrid of nucleic acids which are highly homologous to each other (e.g., a double-stranded polynucleotide which perfectly matches a probe) to (ii) a temperature 15°C lower, preferably 10°C lower, more preferably 5°C lower than the melting temperature (Tm). For example, the stringent conditions can be such that hybridization is carried out, in a common buffer solution for hybridization, at 68°C for 20 hours. In one example, hybridization is carried out, in a buffer solution (0.25 M Na2HPO4; pH 7.2; 7% SDS; 1 mM EDTA; and 1× Denhardt's solution), at 60°C to 68°C, preferably 65°C, more preferably 68°C for 16 hours to 24 hours, and then washing is carried out, in a buffer solution (20 mM Na2HPO4; pH 7.2; 1% SDS; and 1 mM EDTA), at 60°C to 68°C, preferably at 65°C, more preferably at 68°C for 15 minutes. This washing is carried out twice. In another example, prehybridization is carried out overnight at 42°C in a hybridization solution (containing 25% formamide or 50% formamide (for a more stringent condition); 4× SSC (sodium chloride/sodium citrate); 50 mM Hepes pH 7.0; 10× Denhardt's solution; and 20 µg/ml denatured salmon sperm DNA), and then hybridization is carried out by adding a labeled probe thereto and keeping a resulting solution overnight at 42°C. In washing following the hybridization, conditions of a washing solution and a temperature are approximately "1× SSC, 0.1% SDS, 37°C", approximately "0.5× SSC, 0.1% SDS, 42°C" for a more stringent condition, approximately "0.2× SSC, 0.1% SDS, 65°C" for a still more stringent condition. It can be thus expected that as the conditions of the washing following the hybridization become more stringent, DNA having higher homology to a sequence of a probe is isolated. Note, however, that the above combinations of the conditions of the SSC, the SDS, and the temperature are merely examples. A person skilled in the art can achieve stringency similar to the above by appropriately combining the above or other elements (e.g., a probe concentration, a probe length, and a time period for a hybridization reaction) that determine the stringency of hybridization. For example, a person skilled in the art can easily obtain such a gene by referring to Molecular Cloning (Sambrook, J. et al., Molecular Cloning: a Laboratory Manual 2nd ed., Cold Spring Harbor Laboratory Press, 10 Skyline Drive Plainview, NY (1989)).

As used herein, the expression "at least any one of the (first) [...] endogenous gene, the (second) [...] endogenous gene, and the (third) [...] endogenous gene", which specifies one or more of the endogenous genes, means any one of the following 1 through 7. As demonstrated in Examples, the function(s) of one or more of the following 1 through 3 is/are suppressed in an embodiment. In a preferred embodiment, the functions of at least any one of the following 4 through 6 are suppressed. In a more preferred embodiment, the functions of the following 7 are suppressed.
1. The (first) endogenous gene (CCD4-S gene);
2. The (second) endogenous gene (CCD4-T1 gene);
3. The (third) endogenous gene (CCD4-T2 gene);
4. The (first) endogenous gene and the (second) endogenous gene;
5. The (first) endogenous gene and the (third) endogenous gene;
6. The (second) endogenous gene and the (third) endogenous gene; and
7. A combination of the (first) endogenous gene, the (second) endogenous gene, and the (third) endogenous gene.

As used herein, the expression "suppression of a function of a (endogenous) gene" means a state in which a gene that is originally present in a genome does not fulfill its original function. Therefore, the expression "suppression of a function of a (endogenous) gene" encompasses (i) "disruption of a (endogenous) gene", (ii) "a mutation of a (endogenous) gene", and (iii) "suppression of expression of a (endogenous) gene" by a gene (including an exogenous gene) other than the (endogenous) gene.

The "disruption of a (endogenous) gene" means that (i) a gene which is originally present in a genome is not present in the genome or (ii) a transcription product is not produced from a gene which is present in a genome. The "mutation of a (endogenous) gene" means, for example, (i) a mutation of a gene (i.e., decrease or impairment of a function) such that an original functional polypeptide is not produced, (ii) a mutation of a gene such that although a functional polypeptide is produced, the amount of the functional polypeptide produced is decreased, or (iii) a mutation of a gene such that although a functional polypeptide is produced, the stability of the functional polypeptide is decreased. The "suppression of expression of a (endogenous) gene" means, for example, a state in which although no change has occurred to a nucleotide of a (endogenous) gene, the transcriptional or translational function of the gene (from transcription into mRNA to subsequent translation into a polypeptide) is modified through another factor so that (i) the amount of the polypeptide produced is decreased or (ii) no polypeptide is produced. The "suppression of expression of a (endogenous) gene" can occur as a result of, for example, degradation of mRNA which is transcribed from the (endogenous) gene.

As used herein, the "mutation" has the meaning ordinarily understood in the technical field to which the present application belongs, and means, for example, any change in a nucleotide present in a wild-type genome or any change in an amino acid residue present in a wild-type polypeptide (examples of these changes encompass substitution, deletion, insertion, addition, duplication, inversion, and translocation). Therefore, the "mutation of a (endogenous) gene" means, for example, (i) a mutation of a gene such that an original functional polypeptide is not produced (including a mutation such that a polypeptide the function of which is decreased or impaired is produced), (ii) a mutation of a gene such that although a polypeptide is produced, the amount of the polypeptide produced is decreased, (iii) a mutation of a gene such that although a polypeptide is produced, the stability of the polypeptide is decreased, or (iv) a mutation of a gene such that the gene (a coding region or a genomic DNA sequence including an untranslated region) is lost or that transcription from the gene is suppressed (e.g., a transcription-regulating region or a transcription-initiating region is deleted).

In a case where a function is impaired by substitution, the substitution can be present in at least one of the following: a promoter sequence (a sequence upstream (5' end) of a coding region); a sequence downstream (3' end) of the coding region; a 5' untranslated region; a 3' untranslated region; conserved sequences (GT at the 5' end and AG at the 3' end) present at both ends of an intron; and the coding region.

For example, substitution in a nucleotide sequence which is present in a promoter sequence, a 5' untranslated region, or a 3' untranslated region of a gene and which is important in regulating expression of the gene leads to a decrease in the transcriptional activity of the gene or to a decrease in the stability of a transcription product produced from the gene. Any of these decreases can lead to a reduction in transcription product produced from the gene, and ultimately lead to a reduction in translation product. Substitution in a conserved sequence present in an intron (splicing mutation) leads to splicing abnormality of mRNA. This results in abnormal mRNA in which an unnecessary intron is added or inserted. The abnormal mRNA generates an abnormal translation product or translation thereof does not terminate, due to, for example, frame shifting.

In a case where substitution in a nucleotide sequence present in a coding region is a missense mutation, the substitution leads to production of an amino acid different from an original amino acid. This results in a polypeptide the original function of which is decreased or impaired.

Substitution in a coding region can lead to production of a translation product which has an incomplete length or a translation product which does not maintain an original function. The translation product which has an incomplete length is derived from conversion, by the substitution, of a codon which encodes an amino acid into a stop codon (i.e., nonsense mutation). As compared with an original translation product, the translation product which has an incomplete length is such that one or more consecutive amino acid residues including an amino acid residue at a C-terminus are deleted. The nonsense mutation occurs to any codon present upstream of an original stop codon, and is preferably present upstream of the original stop codon with one or more codons therebetween. Thus, a translation product produced from a gene which has a nonsense mutation has an incomplete length. The translation product which does not maintain an original function is produced due to amino acid substitution. In this case, the amount of a transcription product may be equal to that of a transcription product in a wild-type plant. The translation product has, therein, a change in tertiary structure, a decrease in function as a functional domain, or the like. A preferred aspect of the mutation of the present invention is such amino acid substitution that leads to production of a translation product which does not maintain an original function. The amino acid substitution is preferably non-conservative substitution with a high possibility of changing the function of the translation product. Examples of the non-conservative substitution encompass (i) substitution of an amino acid by another amino acid having a different electric charge or different hydrophobicity (e.g., substitution of a basic amino acid by an acidic amino acid, substitution of a basic amino acid or an acidic amino acid by a neutral amino acid, substitution of a neutral amino acid by a basic amino acid or an acidic amino acid, and substitution of a polar amino acid by a non-polar amino acid) and (ii) substitution of an amino acid by another amino acid having a side chain of a different bulk (three-dimensional size).

As another example of a phenomenon caused by a nonsense mutation, in a case where a protein coding region of a CCD4 gene has a nonsense mutation, nonsense-mediated mRNA decay (Brogna and Wen (2009) Nat. Structural Mol. Biol. 16: 107-113) can occur. The nonsense-mediated mRNA decay leads to degradation of a transcription product. Thus, the nonsense mutation may lead to a decrease in the amount of the transcription product. In a case where a target gene is composed of a plurality of exons, it is preferable that at least one exon have the nonsense mutation, and it is more preferable that the exon which has the nonsense mutation be not, among the plurality of exons of which the target gene is composed, an exon which is present most downstream (3' end), in order to cause the nonsense-mediated mRNA decay. A CCD4 gene of a wild-type *Nicotiana* plant has two exons and one intron. A preferred embodiment of the nonsense mutation which leads to the nonsense-mediated mRNA decay is such that at least one nonsense mutation is present in the first exon.

In a case where a mutation(s) (deletion, insertion, and/or the like) other than substitution occur(s) in a promoter sequence, a 5' untranslated region, and/or a 3' untranslated region, a decrease can occur in transcriptional activity or stability as in the case of the substitution, so that (i) the amount of a transcription product can decrease and (ii) the amount of a polypeptide can decrease. In addition, a mutation, other than substitution, in a conserved sequence present in an intron can lead to translation into a polypeptide having an amino acid sequence different from an original amino acid sequence, as in the case of the substitution. A mutation, other than substitution, in a coding region can lead to translation into a polypeptide having an amino acid sequence different from an original amino acid sequence, due to (i) deletion or insertion of an amino acid residue (caused by deletion or insertion of consecutive nucleotides which are multiples of 3) or (ii) frame shifting. Large deletion of an entire gene or insertion of a large fragment into the gene can cause expression itself of the gene to be lost.

An individual which is generated as a result of a mutation or disruption of at least any one of the endogenous genes is herein referred to as a mutant of a tobacco plant (hereinafter simply referred to as "mutant"). Note that a single gene may have a single mutation or a plurality of mutations for causing impairment of a function. Note also that the type(s) of the mutation(s) is/are not limited.

The mutant is preferably in any of states (i) through (iii) below. In the mutant which is in any of the states (i) through (iii), the function of at least one of the endogenous genes is substantially lost, as demonstrated in Examples described later, and the purpose (production of a tobacco plant which shows a change (including an increase and a decrease) in the content of at least one of carotenoids and apocarotenoids, as compared with a wild-type plant) of the mutation or the disruption can be sufficiently and reliably achieved.
(i) Two alleles concerning at least one of the endogenous genes each have the mutation (same or different mutations);
(ii) The two alleles are disrupted; and
(iii) One of the two alleles has the mutation and the other is disrupted.

Suppression of expression of at least any one of the endogenous genes encompass (i) suppression of transcription from the at least any one of the endogenous genes to mRNA, (ii) suppression of translation from the at least any one of the endogenous genes into a polypeptide through mRNA (e.g., degradation of the mRNA) and (iii) suppression of the function of the polypeptide which has been produced by the translation. The degradation of the mRNA can occur due to the nonsense-mediated mRNA decay. The suppression of the transcription can be achieved by, for example, (i) inhibition of a transcription factor which promotes the transcription from the at least any one of the endogenous genes or (ii) inhibition of access of a transcription initiation factor to the at least any one of the endogenous genes. The suppression of the translation can be achieved with use of an antisense RNA molecule, an RNAi molecule, or a co-suppression molecule. The suppression of the function of the polypeptide can be achieved by a molecule which binds to a polypeptide that is functional and thereby inhibits the function of the polypeptide. Examples of such a molecule encompass decoy nucleic acids, ribozymes, antibodies, and inhibitory peptides.

The above suppression (of the transcription, the translation, or the function of the polypeptide) can be achieved by, for example, (i) directly introducing a molecule for achieving the suppression into a plant or (ii) introducing, into a plant, a nucleic acid molecule encoding the molecule (i.e., transformation of the plant). Note, here, that as a result of the transformation of the plant, the nucleic acid molecule is incorporated into any one or more regions of a genome of the plant. Provided that the suppression is achieved, it is unnecessary for the nucleic acid molecule to be incorporated into both an S genome and a T genome as a result of the transformation of the plant.

In the tobacco plant, the suppression of the function is preferably a decrease in the amount of the polypeptide, which is a product of expression of the at least any one of the endogenous genes, as compared with a wild-type plant. Specifically, the amount is decreased through the mutation which leads to the suppression of the function of the at least any one of the endogenous genes which encodes the above wild-type polypeptide.

The polypeptide, which has a sequence identity of 80% or higher with the amino acid sequence represented by SEQ ID NO: 1, 2, or 3, is a polypeptide (or a variant thereof) which is present in a wild-type plant. Since the amount of the polypeptide present in the tobacco plant is decreased as compared with that in a wild-type plant, the tobacco plant is inferior to the wild-type plant in terms of the function. Examples of the function encompass a function of degrading one or more carotenoids.

In the tobacco plant, the suppression of the function is preferably a decrease in the amount of translation into the polypeptide, which is a product of expression of the at least any one of the endogenous genes, as compared with a wild-type plant. The translation into the polypeptide occurs based on (i) a decrease in mRNA (due to, for example, the amount of the mRNA which amount results from the instability of the mRNA itself, promotion of degradation of the mRNA, or suppression of the transcription of the mRNA) or (ii) a decrease in the amount of the translation of the mRNA (due to, for example, lack of elements (tRNA and ribosome) involved in the translation, inhibition of recruit, or functional impairment).

In the tobacco plant, the suppression of the function is preferably a decrease in the amount of the mRNA which has been transcribed from the at least any one of the endogenous genes, as compared with a wild-type plant. The decrease in the amount of the mRNA occurs due to, for example, suppression of the transcription of the mRNA from the at least any one of the endogenous genes. The suppression of the transcription can be achieved by, for example, inhibition of access of a transcription initiation factor to the at least any one of the endogenous genes, which occurs as a result of introduction of the mutation into the at least any one of the endogenous genes.

In the tobacco plant, the suppression of the function is preferably promotion of degradation of the mRNA which has been transcribed from the at least any one of the endogenous genes. The degradation of the mRNA can be caused by, for example, (i) the presence, in the at least any one of the endogenous genes, of a nonsense mutation which causes nonsense-mediated mRNA decay, (ii) the presence of an exogenous factor leading to the degradation of the mRNA, (iii) activation of an endogenous element leading to the degradation of the mRNA, or (iii) the presence of a sequence for promoting the degradation of the mRNA. In a case where the degradation of the mRNA which has been transcribed from the at least any one of the endogenous genes is promoted in the tobacco plant, the mRNA in the tobacco plant is decreased. That is, in the tobacco plant, the suppression of the function may be a decrease in the amount of the mRNA which has been transcribed from the at least any one of the endogenous genes, as compared with a wild-type plant. Note, here, that the expression "decrease in the amount of the mRNA which has been transcribed from the at least any one of the endogenous genes" means that such a transcription product is present at a percentage of 70% or less, 60% or less, 50% or less, 40% or less, 30% or less, 20% or less, 10% or less, 5% or less, or 1% or less with respect to the amount of a transcription product produced from the at least any one of the endogenous genes in a wild-type plant.

In the tobacco plant, the mutation can be insertion, in an outside of a region in which the at least any one of the endogenous genes is present, of a polynucleotide which expresses a factor that promotes the degradation of the mRNA that has been transcribed from the at least any one of the endogenous genes.

The factor is preferably an antisense RNA molecule, an RNAi molecule, or a co-suppression molecule.

In the tobacco plant, the mutation or the disruption of the at least any one of the endogenous genes occurs as a result of, for example, a spontaneous mutation, a mutagen treatment, genetic modification, genome editing, or gene knockout. The spontaneous mutation of the at least any one of the endogenous genes generally occurs due to (i) a replication error and (ii) damage to the at least one of the endogenous genes. The cause of the damage is, for example, exposure to a publicly-known naturally-occurring mutagen (e.g., radiation or ultraviolet rays). The mutagen treatment with respect to the least any one of the endogenous genes can be carried out by artificially causing a mutagen to act on the tobacco plant (as necessary, in combination with suppression of a gene repair function). Examples of the type of the mutagen encompass chemical agents such as ethyl methane sulfonate (EMS), sodium azide, ethidium bromide, and nitrous acid. Note, however, that the mutagen is not limited to these chemical agents, provided that the mutagen is a chemical agent which causes the mutation in a genomic DNA of a *Nicotiana* plant. Examples of the mutagen also encompass γ rays, heavy ion beams, X-rays, neutron rays, and UV rays. Note, however, that the mutagen is not limited to these beams and rays, provided that the mutagen is a radiation or the like which causes the mutation in a genomic DNA of a *Nicotiana* plant. The mutagen is preferably EMS. These methods are preferable from the viewpoint that an exogenous factor need not be added to a target plant. These methods are preferable from the viewpoint that an exogenous factor need not be added to a target plant. Modification of the at least any one of the endogenous genes can be carried out by homologously modifying part or the whole of a target gene with use of a modifying sequence according to a publicly-known genetic modification method. The genome editing of the at least any one of the endogenous genes can be carried out by a publicly-known technique (for example, zinc-finger nucleases (ZFN), transcription activator-like effector nucleases (TALEN), and a CRISPR/Cas9 system). The gene knockout can be carried out by, for example, insertion of a publicly-known transposon (mobile genetic factor) or T-DNA.

The above-described various mutations can be easily introduced into the tobacco plant by a person skilled in the art who has referred to, for example, genome sequences of genes represented by SEQ ID NOs: 18 through 21. Specifically, on the basis of these pieces of sequence information, a region which is present in a genome of any of various tobacco plants encompassed in the scope of the present invention and in which the mutation should be introduced can be determined as appropriate. Note that SEQ ID NO: 18 represents a nucleotide sequence of a CCD4-S gene present in a genome of *Nicotiana tabacum* (Tsukuba 1). SEQ ID NO: 19 represents a nucleotide sequence of a CCD4-T1 gene present in a genome of *Nicotiana tabacum* (Tsukuba 1). SEQ ID NO: 20 represents a nucleotide sequence of a CCD4-T2 gene present in a genome of *Nicotiana tabacum* (Tsukuba 1). SEQ ID NO: 21 represents a nucleotide sequence of a CCD4 gene present in a genome of *Nicotiana sylvestris.* The nucleotide sequences represented by SEQ ID NOs: 18 through 21 each contain a 5' untranslated region and a 3' untranslated region (each about 1 kb).

The tobacco plant is not particularly limited, provided that the tobacco plant is a *Nicotiana* plant. The *Nicotiana* plant is also not particularly limited, provided that the *Nicotiana* plant is a plant belonging to the genus *Nicotiana.* Examples of the tobacco plant encompass *Nicotiana acaulis*, *Nicotiana acuminata, Nicotiana acuminata var. multzjlora*, *Nicotiana africana, Nicotiana alata, Nicotiana amplexicaulis*, *Nicotiana arentsii, Nicotiana attenuata, Nicotiana benavidesii, Nicotiana benthamiana, Nicotiana bigelovii, Nicotiana bonariensis, Nicotiana cavicola, Nicotiana clevelandii*, *Nicotiana cordifolia, Nicotiana corymbosa, Nicotiana debneyi, Nicotiana excelsior, Nicotiana forgetiana, Nicotiana fragrans, Nicotiana glauca, Nicotiana glutinosa, Nicotiana goodspeedii*, *Nicotiana gossei, Nicotiana ingulba, Nicotiana kawakamii, Nicotiana knightiana, Nicotiana langsdorfi, Nicotiana linearis, Nicotiana longiflora, Nicotiana maritima, Nicotiana megalosiphon*, *Nicotiana miersii*, *Nicotiana noctiflora*, *Nicotiana nudicaulis*, *Nicotiana obtusifolia*, *Nicotiana occidentalis*, *Nicotiana occidentalis subsp. Hesperis*, *Nicotiana otophora*, *Nicotiana paniculata*, *Nicotiana pauczjlora*, *Nicotiana petunioides*, *Nicotiana plumbaginifolia*, *Nicotiana quadrivalvis*, *Nicotiana raimondii*, *Nicotiana repanda*, *Nicotiana rosulata*, *Nicotiana rosulata subsp. Ingulba, Nicotiana rotundifolia*, *Nicotiana rustica, Nicotiana setchellii, Nicotiana simulans, Nicotiana solanifolia*, *Nicotiana spegauinii*, *Nicotiana stocktonii*, *Nicotiana suaveolens, Nicotiana sylvestris, Nicotiana tabacum*, *Nicotiana thyrsiflora*, *Nicotiana tomentosa*, *Nicotiana tomentosifomis, Nicotiana trigonophylla*, *Nicotiana umbratica*, *Nicotiana undulata, Nicotiana velutina, Nicotiana wigandioides*, and hybrids of *Nicotiana* plants. Among these *Nicotiana* plants, *Nicotiana benthamiana*, *Nicotiana rustica*, and *Nicotiana tabacum* are more preferable. *Nicotiana rustica* and *Nicotiana tabacum,* each of which is used as a material to produce a tobacco leaf, are particularly preferable.

### [2. Method of producing tobacco plant]

An embodiment of the present invention provides a method of producing a tobacco plant, including the step of introducing a mutation into a genome of a tobacco plant, the mutation causing suppression of a function of at least any one of: an endogenous gene which contains, as a coding region, a polynucleotide that encodes a polypeptide having a sequence identity of 80% or higher with an amino acid sequence represented by SEQ ID NO: 1; an endogenous gene which contains, as a coding region, a polynucleotide that encodes a polypeptide having a sequence identity of 80% or higher with an amino acid sequence represented by SEQ ID NO: 2; and an endogenous gene which contains, as a coding region, a polynucleotide that encodes a polypeptide having a sequence identity of 80% or higher with an amino acid sequence represented by SEQ ID NO: 3.

The step of introducing the mutation causes a change in the content of at least one of carotenoids and apocarotenoids in the tobacco plant through the suppression of the function of the at least any one of the endogenous genes. An outline of the change in the content of at least one of the carotenoids and the apocarotenoids is as described above. Thus, as a specific example of carrying out the above step, introduction of the mutation into the at least any one of the endogenous genes with use of a genome editing technique will be described below. Examples of the genome editing technique which can be used encompass a CRISPR/Cas9 system, TALEN, and ZFN. In the case of the CRISPR/Cas9 system, genome editing can be carried out, if a guide RNA and a Cas9 protein are present in a target cell. In the case of TALEN and ZFN, the genome editing can be carried out, if a fusion protein (in which a DNA-binding domain and a nuclease are fused) is present in the target cell. Therefore, (i) the guide RNA and the Cas9 protein and (ii) the fusion protein can be each directly introduced into the target cell. Examples of a method of directly introducing any of these into the target cell encompass a PEG method, an electroporation method, and a particle bombardment method. Alternatively, a vector in which a construct (including (i) the guide RNA and a polynucleotide which encodes the Cas9 protein and (ii) any promoter and/or any terminator) is inserted may be introduced into the target cell and a tissue via, for example, Agrobacterium.

In the case of the CRISPR/Cas9 system, a sequence which is complementary to a nucleotide sequence present immediately upstream of XGG in a genome forms a base pair with a part of the guide RNA, and a double-stranded genomic DNA is cleaved by Cas9 in the nucleotide sequence. Examples of the nucleotide sequence encompass a part of (i) a polynucleotide (which can have substitution of 0.1% to 1%) encoding a polypeptide having the amino acid sequence represented by SEQ ID NO: 1, 2, or 3 or (ii) a polynucleotide (which can have substitution of 0.1% to 1%) having a nucleotide sequence represented by SEQ ID NO: 4, 5, or 6, which part is 10 or more consecutive bases (e.g., 15 or more consecutive bases, preferably 17 or more consecutive bases, more preferably 18 or more consecutive bases, still more preferably 19 or more consecutive bases, and most preferably 20 or more consecutive bases) present immediately upstream of XGG.

In the case of TALEN, a pair of DNA-binding domains in artificial nucleases forming a dimer each bind to a corresponding one of nucleotide sequences such that each of the nucleotide sequences is present at a terminus of a corresponding one of FokI cleavage domains so as to be away from the terminus by a spacer of 5 to 20 bases. One of the nucleotide sequences is present at one of strands of double-stranded genomic DNA, and the other of the nucleotide sequences is present at the other of the strands of the double-stranded genomic DNA. Therefore, one of the pair of DNA-binding domains binds to one of the strands, and the other of the pair of DNA-binding domains binds to the other of the strands. Each of the DNA-binding domains is composed of repeating units (modules) each of which is composed of 33 to 34 amino acid residues. The number of modules corresponds to the number of nucleotides to which the each of the DNA-binding domains binds. Each of the nucleotide sequences, to each of which a corresponding one of the DNA-binding domains binds, is 10 or more consecutive bases, preferably 14 or more consecutive bases, and more preferably 18 or more consecutive bases, which are to be present at the terminus of a corresponding one of the FokI cleavage domains so as to be away from the terminus by the spacer of 5 to 20 bases and which are a part of (i) a polynucleotide (which can have substitution of 0.1% to 1%) encoding a polypeptide having the amino acid sequence represented by SEQ ID NO: 1, 2, or 3, (ii) a polynucleotide (which can have substitution of 0.1% to 1%) having a nucleotide sequence represented by SEQ ID NO: 4, 5, or 6, or (iii) a polynucleotide forming a strand which is complementary to any of the above polynucleotides.

In the case of ZFN, as in the case of TALEN, a pair of DNA-binding domains in artificial nucleases forming a dimer each bind to a corresponding one of nucleotide sequences such that each of the nucleotide sequences is present at a terminus of a corresponding one of FokI cleavage domains so as to be away from the terminus by a spacer of 5 to 20 bases. Each of the DNA-binding domains is composed of a plurality of zinc finger modules. Each of the nucleotide sequences is 9 or more consecutive bases, preferably 12 or more consecutive bases, and more preferably 18 or more consecutive bases, which are to be present at the terminus of a corresponding one of the FokI cleavage domains so as to be away from the terminus by the spacer of 5 to 20 bases and which are a part of (i) a polynucleotide (which can have substitution of 0.1% to 1%) encoding a polypeptide having the amino acid sequence represented by SEQ ID NO: 1, 2, or 3, (ii) a polynucleotide (which can have substitution of 0.1% to 1%) having a nucleotide sequence represented by SEQ ID NO: 4, 5, or 6, or (iii) a polynucleotide forming a strand which is complementary to any of the above polynucleotides.

The descriptions of the CRISPR/Cas9 system, TALEN, and ZFN and the description of RNAi (described later) can each be read so that, according to the description of each detail, the polypeptide having the amino acid sequence represented by SEQ ID NO: 1, 2, or 3 is replaced with an orthologous polypeptide which (i) has a sequence identity of 80% or higher with the polypeptide and (ii) is present in another species included in *Nicotiana* plants. Likewise, the descriptions in the previous paragraphs can be read so that the polynucleotide having a nucleotide sequence represented by SEQ ID NO: 4, 5, or 6 is replaced with a polynucleotide of an orthologous gene which polynucleotide (i) has a sequence identity of 80% or higher with the polynucleotide and (ii) is present in another species included in *Nicotiana* plants.

As has been described above, the mutation, which causes the suppression of the function of the at least any one of the endogenous genes and which is introduced into the tobacco plant, is preferably genetically inherited. However, an exogenous polynucleotide which has been introduced into the tobacco plant for genome editing is preferably eliminated from the tobacco plant after it is confirmed that a desired mutation is introduced in the tobacco plant. In a case where the exogenous polynucleotide is retained in the tobacco plant, an undesired mutation may (continue to) be introduced. This may cause a desired character (a change in the content of at least one of the carotenoids and the apocarotenoids) to be lost.

The introduction of the mutation into the at least any one of the endogenous genes of the tobacco plant or disruption of the at least any one of the endogenous genes of the tobacco plant can be achieved through another biotechnological method (e.g., a method in which transposon or Agrobacterium is employed). Specific examples of the method encompass a method in which retrotransposon tnt1 of tobacco, transposon of another plant, or T-DNA of a Ti plasmid of Agrobacterium is introduced into the tobacco plant.

Alternatively, the introduction or the disruption can be achieved through another method (mutagen treatment of the tobacco plant). Examples of a mutagen encompass small-molecule compounds (such as ethyl methane sulfonate (EMS), N-ethyl-N-nitrosourea (ENU), and sodium azide) and radiations (such as γ rays, heavy ion beams, X-rays, neutron beams, and ultraviolet rays).

The mutation can be introduced into any regenerable tobacco plant. Examples of the tobacco plant encompass seeds, roots, leaves, flowers, reproductive organs, and embryos. The tobacco plant is preferably a seed.

What can be obtained by any of the above methods can be a mutant population of a plant which has various mutations (or no mutation). Therefore, an individual exhibiting a desired phenotype can be further selected from the mutant population. As an example of the selection of the individual, the following description will discuss a procedure for selecting a desired individual from a mutant population (panel) which is obtained in a case where a treatment is carried out with use of a mutagen.

A tobacco mutant the function of which is impaired due to mutations in the total of four alleles in both a T genome and an S genome can be obtained by, for example, the following method. A tobacco plant is treated with use of a mutagen as has been described to prepare a population (panel) of tobacco mutants each having mutations in the whole tobacco genome. Subsequently, genomic DNAs are extracted. With use of gene-specific primers in the S genome and the T genome, target genes (polynucleotides) are amplified from the genomic DNAs of the panel. Thereafter, nucleotide sequences of resulting products are determined, and lines having homozygous mutations are then selected. First, a line (M2) having a homozygous mutation in an S genome and a line (M2) having a homozygous mutation in a T genome are obtained and then crossed to prepare F1 individuals. Subsequently, selfed progeny (F2) is developed from the F1 individuals. From the selfed progeny (F2), a line having homozygous mutations in both an S genome and a T genome is obtained (such a line is obtained at a probability of 1/16 since two elements are recessive).

A tobacco mutant the function of which is impaired due to mutations in the total of six alleles at two loci in a T genome and at one locus in an S genome can be obtained by, for example, the following method. A line having homozygous mutations in both an S genome and a T genome, which line has been obtained as described above, is crossed with a line having a homozygous mutation at the other allele in a T genome to prepare F1 individuals. Subsequently, selfed progeny (F2) is developed from the F1 individuals. From the selfed progeny (F2), a line having homozygous mutations in an S, a T, and the other T is obtained (such a line is obtained at a probability of 1/64 since three elements are recessive).

The other examples of carrying out the step of introducing the mutation encompass suppression of expression of the at least any one of the genes and introduction of the mutation into the at least any one of the genes, which are performed through transformation of the tobacco plant with use of a vector.

The vector used to transform the tobacco plant for the purpose of suppression of expression of the at least any one of the genes or introduction of the mutation into the at least any one of the genes is not limited to any particular one, provided that a polynucleotide which is inserted in the vector can be expressed in a plant cell. Suitable examples of the vector encompass pBI, pPZP, and pSMA vectors each of which allows introduction of a target polynucleotide into a plant cell via Agrobacterium. In particular, plasmids of binary vectors (e.g., pBIG, pBIN19, pBI101, pBI121, pBI221, and pPZP202) are preferable.

In a case where suppression of expression of the at least any one of the genes is achieved by RNAi, a trigger sequence, which is used by RNAi to suppress expression of a target gene, is inserted into the vector. Examples of the trigger sequence encompass (i) a polynucleotide (sense RNA portion) which is (a) a part of a polynucleotide (which can have substitution of 0.1% to 1%) encoding a polypeptide having the amino acid sequence represented by SEQ ID NO: 1, 2, or 3 or a part of a polynucleotide (which can have substitution of 0.1% to 1%) having a nucleotide sequence represented by SEQ ID NO: 4, 5, or 6 and (b) represented by a nucleotide sequence of at least 21 to 30 consecutive bases (e.g., 21 or more bases, 22 or more bases, 23 or more bases, 24 or more bases, 25 or more bases, 26 or more bases, 27 or more bases, 28 or more bases, 29 or more bases, and 30 or more bases) and (ii) a polynucleotide (antisense RNA portion) which is represented by a nucleotide sequence that is complementary to the polynucleotide (i). More specifically, the nucleotide sequence of the "at least 21 to 30 consecutive bases" described above means a nucleotide sequence of 21 or more consecutive bases, 23 or more consecutive bases, 25 or more consecutive bases, 30 or more consecutive bases, 35 or more consecutive bases, 40 or more consecutive bases, 45 or more consecutive bases, 50 or more consecutive bases, 60 or more consecutive bases, 70 or more consecutive bases, 80 or more consecutive bases, 90 or more consecutive bases, or 100 or more consecutive bases.

As has been described, suppression of expression of the at lease any one of the endogenous genes in the tobacco plant is preferably genetically inherited. Therefore, the trigger sequence is preferably incorporated in a genome of the tobacco plant.

The tobacco plant in which expression of two or more of the endogenous genes is simultaneously suppressed can be obtained by crossing two tobacco plants in which expression of respective different ones of the endogenous genes is suppressed. Alternatively, the tobacco plant in which expression of two or more of the endogenous genes is simultaneously suppressed can be obtained by (i) performing transformation which can cause expression of different ones of the endogenous genes to be simultaneously suppressed and then (ii) selecting the tobacco plant in which expression of different ones of the endogenous genes is simultaneously suppressed.

Note that in a case where the tobacco plant in which the functions of two or more of the endogenous genes are suppressed is obtained by crossing, (i) one of tobacco plants to be crossed can be prepared by a mutation or disruption of at least any one of the endogenous genes and (ii) the other one of the tobacco plants to be crossed can be prepared by suppression of expression of at least any one of the endogenous genes which suppression is caused by transformation.

The mutation or disruption of the at least any one of the endogenous genes can be determined by identifying the presence/absence of the mutation in the at least any one of the endogenous genes. A method of identifying the mutation in the at least any one of the endogenous genes only needs to allow the determination of the presence/absence of the mutation. Examples of the method encompass (1) a method in which a commercially available DNA sequence having the mutation is amplified by PCR or the like, and then a DNA nucleotide sequence is directly decoded with use of a sequencer or the like, (2) a method in which a difference in sequence is detected by a difference in distance of electrophoresis by a single strand conformation polymorphism (SSCP) method, (3) a method in which single nucleotide polymorphism (SNP) is detected by a CycleavePCR method, (4) a method in which the presence/absence of the mutation is identified by cleaving a mismatch site(s) with use of T7 EndonucleaseI or the like, (5) a cleaved amplified polymorphic sequence (CAPS) method in which the presence/absence of the mutation can be determined by the presence/absence of cleavage by a restriction enzyme treatment, (6) a derived CAPS (dCAPS) method in which a set of primers including a mismatch intentionally is used so that the presence/absence of the mutation can be determined by the presence/absence of cleavage by restriction enzymes, (7) a method (e.g., a PCR method in which a TaqMan probe is used, and MassARRAY analysis) in which the presence/absence of the mutation is determined by identifying, with use of a probe which specifically hybridizes to a mutant sequence, whether or not the probe is hybridized, and (8) a method in which, in a case where the mutation is deletion or insertion, the mutation is identified by a difference in mobility of electrophoresis. Alternatively, the mutation in the at least any one of the genes can be determined by comparing (i) the size or the expression level of a polypeptide which results from modification of the at least any one of the genes with (ii) that of a wild-type protein. Specifically, such a comparison can be made by carrying out, for example, a Western blotting method.

### [3. Others]

Another aspect of the present invention provides a method of regulating the contents of apocarotenoids in a tobacco plant. The method includes the following steps (a) through (c).
(a) The step of regulating expression or activity of a carotenoid cleavage dioxygenase in the tobacco plant, the carotenoid cleavage dioxygenase containing one of the following (i) through (iii):
   (i) a polynucleotide which encodes the carotenoid cleavage dioxygenase and which includes, consists of, or essentially consists of a sequence having a sequence identity of at least 80% with a polynucleotide represented by SEQ ID NO: 4, 5 or 6;
   (ii) a polypeptide which is encoded by the polynucleotide (i); and
   (iii) a polypeptide having a sequence identity of at least 80% with SEQ ID NO: 1, 2 or 3 or a sequence identity of at least 80% with SEQ ID NO: 7.
(b) The step of measuring the contents of apocarotenoids in at least a part of a mutant plant, an unnatural plant, or a transgenic plant obtained in the step (a) or the contents of apocarotenoids in aerosol generated during burning or heating of the at least the part of the mutant plant, the unnatural plant, or the transgenic plant.
(c) The step of identifying the mutant plant, the unnatural plant, or the transgenic plant which has changed apocarotenoid contents as compared with a control plant in which expression or activity of the carotenoid cleavage dioxygenase is not regulated.

In the above method, the apocarotenoids can be β-ionone and dihydroactinidiolide, and regulating the contents can be increasing the contents. In the above method, the apocarotenoids can be β-damascenone, megastigmatrienone (and structural isomers thereof), and 3-hydroxy-β-damascone, and regulating the contents can be decreasing the contents.

An embodiment of the present invention provides a tobacco leaf of the above tobacco plant and a cured leaf (cured tobacco) obtained from the tobacco leaf. The cured leaf is obtained by curing the tobacco leaf. As a curing method, any method can be employed. The curing method can be, but is not limited to, air curing, warm-air curing, flue curing, or the like, for example. Note that, in this specification, the cured leaf encompasses cut fillers, powders, sheets, stems, granules, and extracts each of which is obtained from the cured leaf.

An embodiment of the present invention provides a tobacco product in which the cured leaf is used. The tobacco product can be in any form. The tobacco product can be, but is not limited to, shred tobaccos, cigars, pipe smoking tobaccos, cigarettes, electronic cigarettes, hookah tobaccos, snuff tobaccos (including snus and snuff), heat-not-burn tobacco products (using, as an aerosol source, aerosol generated by heating of tobacco), non-heated tobacco products (for inhaling a flavor of tobacco without heating the tobacco), or the like, for example.

### (Summary)

With the above embodiments considered together, the present invention can be summarized as follows.
(1) A tobacco plant in which a mutation is introduced in a genome, the mutation causing suppression of a function of at least any one of:
   an endogenous gene which contains, as a coding region, a polynucleotide that encodes a polypeptide having a sequence identity of 80% or higher with an amino acid sequence represented by SEQ ID NO: 1;
   an endogenous gene which contains, as a coding region, a polynucleotide that encodes a polypeptide having a sequence identity of 80% or higher with an amino acid sequence represented by SEQ ID NO: 2; and
   an endogenous gene which contains, as a coding region, a polynucleotide that encodes a polypeptide having a sequence identity of 80% or higher with an amino acid sequence represented by SEQ ID NO: 3.
(2) The tobacco plant as described in (1), wherein the suppression of the function is a decrease in an amount of the polypeptide, as compared with a wild-type plant.
(3) The tobacco plant as described in (2), wherein the suppression of the function is a decrease in an amount of translation into the polypeptide, as compared with a wild-type plant.
(4) The tobacco plant as described in (2), wherein the suppression of the function is a decrease in an amount of transcription of mRNA from the at least any one of the endogenous genes, as compared with a wild-type plant.
(5) The tobacco plant as described in (2), wherein the suppression of the function is promotion of degradation of mRNA which has been transcribed from the at least any one of the endogenous genes.
(6) The tobacco plant as described in any one of (1) through (5), wherein the mutation is introduced in the at least any one of the endogenous genes.
(7) The tobacco plant as described in (6), wherein the mutation is introduced by spontaneous mutation, mutagen treatment, genome editing, or gene knockout.
(8) The tobacco plant as described in (5), wherein the mutation is insertion, in an outside of a region in which the at least any one of the endogenous genes is present, of a polynucleotide which expresses a factor that promotes the degradation of the mRNA.
(9) The tobacco plant as described in (8), wherein the factor is an antisense RNA molecule, an RNAi molecule, or a co-suppression molecule.
(10) The tobacco plant as described in any one of (1) through (9), wherein the tobacco plant belongs to *Nicotiana tabacum* or *Nicotiana rustica.*
(11) A method of producing a tobacco plant, including the step of introducing a mutation into a genome of a tobacco plant, the mutation causing suppression of a function of at least any one of:
   an endogenous gene which contains, as a coding region, a polynucleotide that encodes a polypeptide having a sequence identity of 80% or higher with an amino acid sequence represented by SEQ ID NO: 1;
   an endogenous gene which contains, as a coding region, a polynucleotide that encodes a polypeptide having a sequence identity of 80% or higher with an amino acid sequence represented by SEQ ID NO: 2; and
   an endogenous gene which contains, as a coding region, a polynucleotide that encodes a polypeptide having a sequence identity of 80% or higher with an amino acid sequence represented by SEQ ID NO: 3.
(12) The method as described in (11), wherein the suppression of the function is a decrease in an amount of the polypeptide, as compared with a wild-type plant.
(13) The method as described in (12), wherein the suppression of the function is a decrease in an amount of translation into the polypeptide, as compared with a wild-type plant.
(14) The method as described in (12), wherein the suppression of the function is a decrease in an amount of transcription of mRNA from the at least any one of the endogenous genes, as compared with a wild-type plant.
(15) The method as described in (12), wherein the suppression of the function is promotion of degradation of mRNA which has been transcribed from the at least any one of the endogenous genes.
(16) The method as described in any one of (12) through (15), wherein the step of introducing the mutation includes introducing the mutation into the at least any one of the endogenous genes.
(17) The method as described in (16), wherein the step of introducing the mutation is carried out by spontaneous mutation, mutagen treatment, genetic modification, genome editing, or gene knockout.
(18) The method as described in any one of (12) through (15), wherein the step of introducing the mutation includes inserting, into an outside of a region in which the at least any one of the endogenous genes is present, a polynucleotide which expresses a factor that promotes the degradation of the mRNA having been transcribed from the at least any one of the endogenous genes.
(19) The method as described in (18), wherein the factor is an antisense RNA molecule, an RNAi molecule, or a co-suppression molecule.
(20) Offspring or bred progeny, the offspring being of a tobacco plant described in any one of (1) through (10) or a tobacco plant produced by a method described in any one of (11) through (19), the bred progeny being obtained by crossing a tobacco plant described in any one of (1) through (10) or a tobacco plant produced by a method described in any one of (11) through (19) with another tobacco plant.
(21) A tobacco leaf harvested from a tobacco plant described in any one of (1) through (10), a tobacco plant produced by a method described in any one of (11) through (19), or offspring or bred progeny described in (20).
(22) A cured leaf produced from a tobacco leaf described in (21).
(23) A tobacco product containing a cured leaf described in (22).

The above embodiments can be alternatively summarized as follows.
(1) A tobacco plant in which a mutation is introduced in a genome, the mutation causing suppression of a function of at least any one of:
   an endogenous gene which contains, as a coding region, a polynucleotide that encodes a polypeptide having a sequence identity of 80% or higher with an amino acid sequence represented by SEQ ID NO: 1;
   an endogenous gene which contains, as a coding region, a polynucleotide that encodes a polypeptide having a sequence identity of 80% or higher with an amino acid sequence represented by SEQ ID NO: 2; and
   an endogenous gene which contains, as a coding region, a polynucleotide that encodes a polypeptide having a sequence identity of 80% or higher with an amino acid sequence represented by SEQ ID NO: 3.
(2) The tobacco plant as described in (1), wherein the suppression of the function is a decrease in an amount of mRNA which has been transcribed from the at least any one of the endogenous genes, as compared with a wild-type plant.
(3) The tobacco plant as described in (1) or (2), wherein the suppression of the function is promotion of degradation of mRNA which has been transcribed from the at least any one of the endogenous genes.
(4) The tobacco plant as described in any one of (1) through (3), wherein the mutation is introduced in the at least any one of the endogenous genes.
(5) The tobacco plant as described in (4), wherein the mutation is introduced by mutagen treatment, genome editing, or gene knockout.
(6) The tobacco plant as described in (5), wherein the mutation is introduced by the mutagen treatment.
(7) The tobacco plant as described in (3), wherein the mutation is insertion, in an outside of a region in which the at least any one of the endogenous genes is present, of a polynucleotide which expresses a factor that promotes the degradation of the mRNA.
(8) The tobacco plant as described in (7), wherein the factor is an antisense RNA molecule, an RNAi molecule, or a co-suppression molecule.
(9) The tobacco plant as described in any one of (1) through (8), wherein the suppression of the function is a decrease in an amount of the polypeptide which has an original function, as compared with a wild-type plant.
(10) The tobacco plant as described in (9), wherein the suppression of the function is a decrease in an amount of translation into the polypeptide which has the original function, as compared with a wild-type plant.
(11) The tobacco plant as described in any one of (1) through (10), wherein the tobacco plant belongs to *Nicotiana tabacum, Nicotiana rustica,* or *Nicotiana sylvestris.*
(12) A method of producing a tobacco plant, including the step of introducing a mutation into a genome of a tobacco plant, the mutation causing suppression of a function of at least any one of:
   an endogenous gene which contains, as a coding region, a polynucleotide that encodes a polypeptide having a sequence identity of 80% or higher with an amino acid sequence represented by SEQ ID NO: 1;
   an endogenous gene which contains, as a coding region, a polynucleotide that encodes a polypeptide having a sequence identity of 80% or higher with an amino acid sequence represented by SEQ ID NO: 2; and
   an endogenous gene which contains, as a coding region, a polynucleotide that encodes a polypeptide having a sequence identity of 80% or higher with an amino acid sequence represented by SEQ ID NO: 3.
(13) The method as described in (12), wherein the suppression of the function is a decrease in an amount of mRNA which has been transcribed from the at least any one of the endogenous genes, as compared with a wild-type plant.
(14) The method as described in (12) or (13), wherein the suppression of the function is promotion of degradation of mRNA which has been transcribed from the at least any one of the endogenous genes.
(15) The method as described in any one of (12) through (14), wherein the step of introducing the mutation includes introducing the mutation into the at least any one of the endogenous genes.
(16) The method as described in (15), wherein the step of introducing the mutation is carried out by mutagen treatment, genome editing, or gene knockout.
(17) The method as described in (16), wherein the mutation is introduced by the mutagen treatment.
(18) The method as described in any one of (12) through (15), wherein the step of introducing the mutation includes inserting, into an outside of a region in which the at least any one of the endogenous genes is present, a polynucleotide which expresses a factor that promotes the degradation of the mRNA having been transcribed from the at least any one of the endogenous genes.
(19) The method as described in (18), wherein the factor is an antisense RNA molecule, an RNAi molecule, or a co-suppression molecule.
(20) The method as described in any one of (12) through (19), wherein the suppression of the function is a decrease in an amount of the polypeptide which has an original function, as compared with a wild-type plant.
(21) The method as described in (20), wherein the suppression of the function is a decrease in an amount of translation into the polypeptide which has the original function, as compared with a wild-type plant.
(22) Offspring or bred progeny, the offspring being of a tobacco plant described in any one of (1) through (11) or a tobacco plant produced by a method described in any one of (12) through (21), the bred progeny being obtained by crossing a tobacco plant described in any one of (1) through (11) or a tobacco plant produced by a method described in any one of (12) through (21) with another tobacco plant.
(23) A tobacco leaf which is obtained from a tobacco plant,
   in the tobacco leaf, a mutation being introduced in a genome, the mutation causing suppression of a function of at least any one of:
   an endogenous gene which contains, as a coding region, a polynucleotide that encodes a polypeptide having a sequence identity of 80% or higher with an amino acid sequence represented by SEQ ID NO: 1;
   an endogenous gene which contains, as a coding region, a polynucleotide that encodes a polypeptide having a sequence identity of 80% or higher with an amino acid sequence represented by SEQ ID NO: 2; and
   an endogenous gene which contains, as a coding region, a polynucleotide that encodes a polypeptide having a sequence identity of 80% or higher with an amino acid sequence represented by SEQ ID NO: 3,
   the tobacco leaf having a higher total carotenoid content than a tobacco leaf which is obtained from a wild-type tobacco plant.
(24) A cured leaf which is obtained from a tobacco plant,
   in the cured leaf, a mutation being introduced in a genome, the mutation causing suppression of a function of at least any one of:
   an endogenous gene which contains, as a coding region, a polynucleotide that encodes a polypeptide having a sequence identity of 80% or higher with an amino acid sequence represented by SEQ ID NO: 1;
   an endogenous gene which contains, as a coding region, a polynucleotide that encodes a polypeptide having a sequence identity of 80% or higher with an amino acid sequence represented by SEQ ID NO: 2; and
   an endogenous gene which contains, as a coding region, a polynucleotide that encodes a polypeptide having a sequence identity of 80% or higher with an amino acid sequence represented by SEQ ID NO: 3,
   the cured leaf having a higher total carotenoid content than a cured leaf which is obtained from a wild-type tobacco plant.
(25) A tobacco product containing a cured leaf described in (24).

The following description will discuss details of the embodiment of the present invention with reference to Examples. The present invention is, of course, not limited to the Examples below and particulars can have various aspects. Further, the present invention is not limited to the embodiments, but can be altered by a person skilled person in the art within the scope of the claims. An embodiment derived from a proper combination of technical means disclosed in respective different embodiments is also encompassed in the technical scope of the present invention. Moreover, all the literatures described in this specification are hereby incorporated by reference.

### Examples

### [1. Change occurring in tobacco plant due to suppression of expression of CCD4 gene]

Recombinants in each of which expression of CCD4 genes was suppressed (hereinafter simply referred to as "recombinants") were prepared, and a change occurring in each of tobacco plants due to suppression of the expression of the CCD4 genes was identified.

### (1a) Preparation for transformation

In order to prepare the recombinants, a vector for transformation was first prepared as described below.

A region (323 bp) which had a high identity with SEQ ID NOs: 4 through 6 was selected, as a RNAi trigger sequence for suppressing expression of all three CCD4 genes (a CCD4-S gene, a CCD4-T1 gene, and a CCD4-T2 gene) in *Nicotiana tabacum.* The region was amplified by PCR with use of PrimeSTAR (registered trademark) Max DNA Polymerase (Takara-Bio Inc.), while cDNA of the CCD4-T2 gene was used as a template. The conditions of the PCR and primers were as follows. Note that CACC which did not match SEQ ID NOs: 4 through 6 was added to the 5' end of SEQ ID NO: 9.

### (Conditions of PCR)

94°C for 30 seconds
30 cycles to 40 cycles of 98°C for 10 seconds, 55°C for 5 seconds, and then 72°C for 10 seconds
72°C for 10 seconds

### (Primer set)

Forward primer NtCCD4-F1: 5'-CACCGCGTACATCCGAAATGGCCC-3' (SEQ ID NO: 9)
Reverse primer NtCCD4-R1: 5'-AGTTTGCCTCCGAATAAAGC-3' (SEQ ID NO: 10).

A resulting PCR product was cloned into a pENTR (trademark)/D-TOPO (trademark) vector (Thermo Fisher Scientific Inc.), and then the nucleotide sequence of the RNAi trigger sequence (SEQ ID NO: 11) was confirmed. Thereafter, the RNAi trigger sequence was introduced into a pSP231 vector with use of Gateway (trademark) LR Clonase (trademark) II Enzyme Mix (Thermo Fisher Scientific Inc.). In order to confirm the sequence thus introduced, the sense strand and the antisense strand of the RNAi trigger sequence introduced into the pSP231 vector were individually amplified by PCR, and then the nucleotide sequence of each of the sense strand and the antisense strand was confirmed. The pSP231 vector is a binary vector which (i) has a green fluorescent protein gene (GFP) expression cassette that is inserted in a SacI site of pHellsgate12 (see the literature: Wesley et al., 2001, Plant J., 27, 581-590), (ii) has an inverted repeat sequence of a trigger sequence that is flanked with pdk/cat introns, and (iii) is capable of expressing an RNAi trigger sequence with use of a cauliflower mosaic virus 35S RNA gene promoter. With use of the pSP231 vector containing the RNAi trigger sequence, Agrobacterium (*Agrobacterium tumefaciens*) LBA4404 was transformed by electroporation. In a resulting transformed Agrobacterium, the presence of the RNAi trigger sequence was confirmed by PCR, and then the Agrobacterium was used for transformation of tobacco.

### (1b) Preparation of recombinants

With use of each of four varieties (Petit Havana SR-1, Tsukuba 1, K326, and Coker319) of *Nicotiana tabacum,* transformation of tobacco was carried out as follows by a general method. A section of a tobacco leaf was infected with the transformed Agrobacterium, and was cultured in a Linsmaier and Skoog medium containing kanamycin (50 µg/ml) to obtain calluses. From the calluses, redifferentiated individuals which were kanamycin-resistant were obtained. From these redifferentiated individuals, individuals in each of which GFP fluorescence was observed in the entire leaves thereof were selected. The individuals thus selected (T0 individuals) were transplanted to 9-cm pots, and were grown under fixed conditions in a containment greenhouse at 23°C to 25°C. On the basis of the results of a real-time RT-PCR expression analysis, lines in each of which the expression of the CCD4 genes was decreased by approximately 20% as compared with a non-transformant (Petit Havana SR-1, Tsukuba 1, K326, or Coker319 of an original variety) were selected as recombinants. The recombinants prepared from each of the above four varieties each showed a phenotype different from that of a corresponding one of the original varieties (i.e., the lower leaves of the recombinants were clearly yellowed).

### (1c) Quantitative analysis of carotenoid

Five to six lower leaves harvested from each of (i) three lines (RNAi-1-3, 1-8, and 1-15), out of the above recombinants (T0 generation) prepared from Petit Havana SR-1, and (ii) two individuals of the original variety were freeze-dried and then ground to obtain a sample. The sample was subjected to a lutein (carotenoid) analysis. Into a 30-mL screw cap glass centrifuge tube, 1.0 g of the sample and then 10 mL of an extraction solution (acetone : ethanol = 1:1) were introduced. After the centrifuge tube was capped with a screw cap, ultrasonic extraction was carried out for 30 minutes. After the ultrasonic extraction, the centrifuge tube was allowed to stand for 20 minutes, and then subjected to centrifugal separation (3000 rpm, 10 min). A resulting supernatant was filtered through a PTFE filter (pore diameter: 0.45 µm), and a filtrate was subjected to a liquid chromatographic analysis. Devices, an instrument, and conditions employed in the analysis are as follows.
· High-performance liquid chromatograph: LC-2000Plus (JASCO Corporation, Tokyo)
· PDA detector: MD-2010 Plus (JASCO Corporation, Tokyo)
· Column: Develosil (registered trademark) (ODS-HG-5, inner diameter: 4.6 mm, length: 250 mm, Nomura Chemical Co., Ltd., Aichi)
· Column oven temperature: 40°C
· Eluent flow rate: 1.0 mL/min
· Eluent A: methanol
· Eluent B: tetrahydrofuran (containing no stabilizer)
· Elution condition: isocratic solvent elution (eluent A : eluent B = 9:1)
· Injection amount: 10 µL
· PDA detection wavelength: 455 nm

The content of lutein per dry weight was calculated by a calibration curve method in which the peak area value of an absorption spectrum detected at an optical wavelength of 455 nm was used. A calibration curve (exhibiting linearity in the range of 1 µg/mL to 20 µg/mL with a coefficient of correlation of 0.9999) created on the basis of the results of analyzing 1 µg/mL, 5 µg/mL, 10 µg/mL, and 20 µg/mL of lutein standard solutions with use of a liquid chromatograph was used. Table 2 shows results. As shown in Table 2, the three lines (RNAi-1-3, 1-8, and 1-15) each contained approximately three times as much lutein (major carotenoid) as the two individuals of the original variety (Controls 1 and 2).

**[Table 2]**

| | lutein (µg/g dry weight) |
|---|---|
| Control 1 | 128.0 |
| Control 2 | 142.7 |
| RNAi-1-3 | 418.2 |
| RNAi-1-8 | 429.1 |
| RNAi-1-15 | 442.3 |

As described above, suppression of the expression of the CCD4 genes in the tobacco plants caused accumulation of the carotenoid in leaves thereof. Note that it is reported that, in potato, which is another solanaceous plant, although CCD4 genes are highly expressed in leaves thereof, downward regulation of expression of the CCD4 genes does not affect accumulation of carotenoids in the leaves (Campbell et al., Plant Physiology, 2010, Vol. 154, pp. 656-664).

### [2. Preparation of tobacco plants each having mutation in CCD4 gene]

In order to obtain other tobacco plants in each of which the above-described change occurred, tobacco plants each having a mutation(s) in a CCD4 gene(s) were prepared.

Lines each having a mutation in a CCD4-S gene or a CCD4-T1 gene were selected from an EMS mutant population (M2 generation, approximately 2000 lines) of a tobacco cultivar (Tsukuba 1) in accordance with sequence determination by amplicon sequencing. As a line having a mutation in the CCD4-S gene, CCD4-s-1 (0844 line) was obtained in which one nonsense mutation (a change of a codon encoding the 72nd glutamine (Q) of a CCD4-S protein to a stop codon by nucleotide substitution) homozygously occurred in the CCD4-S gene. As lines each having a mutation in the CCD4-T1 gene, two lines (CCD4-t1-1: 0283 line and CCD4-t1-2: 0135 line) were obtained in each of which one nonsense mutation homozygously occurred in the CCD4-T1 gene. In the CCD4-t1-1, a codon encoding the 46th glutamine (Q) of a CCD4T1 protein changed to a stop codon by nucleotide substitution. In the CCD4-t1-2, a codon encoding the 187th arginine (R) of a CCD4T1 protein changed to a stop codon by nucleotide substitution.

The above three lines were used to prepare, in the following manner, tobacco plants (double mutants) each having mutations in two CCD4 genes. An F1 plant obtained by crossing CCD4-s-1 with CCD4-t1-1 via artificial pollination was selfed to obtain an F2-generation population. From the F2-generation population, a line (CCD4-st1-1) which homozygously had a mutation in each of two CCD4 genes and a line (WT1) in which the two CCD4 genes were homozygously of a wild-type were selected in accordance with sequence determination by amplicon sequencing. An F1 plant obtained by crossing CCD4-s-1 with CCD4-t1-2 via artificial pollination was selfed to obtain an F2-generation population. From the F2-generation population, a segregant line (CCD4-st1-2) which homozygously had a mutation in each of two CCD4 genes and a line (WT2) in which the two CCD4 genes were homozygously of a wild type were selected in accordance with sequence determination by amplicon sequencing. Note that the lines (WT1 and WT2) in each of which the two CCD4 genes were homozygously of a wild-type and each of which was a control for a corresponding one of the double mutants will be referred to as "homozygous wild-type tobacco plants".

Lines each having a mutation in a CCD4 gene were selected from an EMS mutant population (M2 generation, approximately 4000 lines) of a tobacco wild species (*Nicotiana sylvestris*) in accordance with sequence determination by amplicon sequencing. Note that *Nicotiana sylvestris* has only one CCD4 gene in a genome. As the lines each having a mutation in the CCD4 gene, two lines (08N-465 line and 06N-7039 line) were obtained in each of which one nonsense mutation homozygously occurred in the CCD4 gene. In the 08N-465 line, a codon encoding the 72th glutamine (Q) of a CCD4 protein changed to a stop codon by nucleotide substitution. In the 06N-7039 line, a codon encoding the 150th arginine (R) of a CCD4 protein changed to a stop codon by nucleotide substitution.

Primers used for the above amplicon sequencing are shown below.
Tobacco CCD4-S forward primer: TCATCTTCTCCTTCTCTTAAA (SEQ ID NO: 12)
Tobacco CCD4-S reverse primer: CGGAGAATACATTTGGCAA (SEQ ID NO: 13)
Tobacco CCD4-T1 forward primer: TCATCTTCTCTTGCTCTTAAG (SEQ ID NO: 14)
Tobacco CCD4-T1 reverse primer: CAGAGAATACATTTGGGAT (SEQ ID NO: 15)
*Nicotiana sylvestris* CCD4 forward primer: CCTTTCTACATTATCACAACACCCTA (SEQ ID NO: 16)
*Nicotiana sylvestris* CCD4 reverse primer: TCACCATCTGGGGCTAATTT (SEQ ID NO: 17)

Note that CCD4-st1-1 and CCD4-st1-2 each of which was developed in a greenhouse each exhibited a phenotype (yellowing of lower leaves) which was not seen in CCD4-s-1, CCD4-t1-1, CCD4-t1-2, WT1, and WT2. The phenotype was very similar to that exhibited by the recombinants of Example 1. Further, the 08N-465 and 06N-7039 lines each exhibited the phenotype (yellowing of lower leaves) which was not seen in *Nicotiana sylvestris,* which was of a wild-type.

### [3. Quantitative analyses of carotenoids and apocarotenoids]

### (3a) Major carotenoid contents of double mutants grown in greenhouse

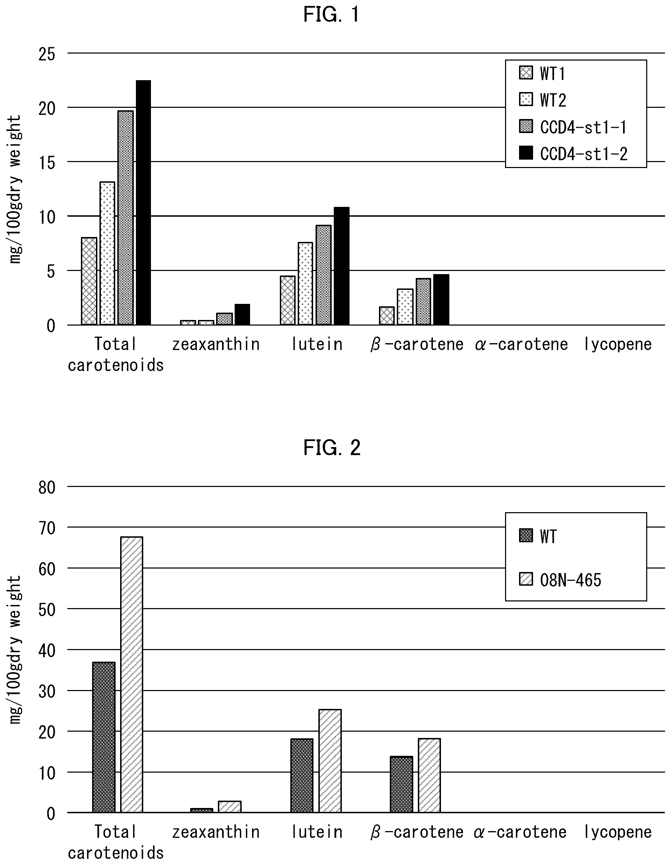

Two individuals in each of two lines of double mutants (CCD4-st1-1 and CCD4-st1-2) and two lines of homozygous wild-type tobacco plants (WT1 and WT2), which double mutants and homozygous wild-type tobacco plants had been obtained in Example 2, were grown in a greenhouse at 23°C to 25°C. Seven to seventeen days after topping, four to five lower leaves harvested from each of the individuals (eight to ten leaves per line) were frozen with use of liquid nitrogen, and stored at -80°C. Thereafter, the lower leaves were further freeze-dried and ground. Then, a mixture of the lower leaves harvested from two individuals in the same line was used as one sample. An analysis of the major carotenoid contents of each of samples was requested to Japan Food Research Laboratories. The results received from Japan Food Research Laboratories are summarized in Fig. 1. The item "Total carotenoids" in Fig. 1 is based on a value obtained by actually measuring the total carotenoid content of each of the samples by absorption photometry (absorption coefficient of lutein: E^{1%} 1 cm = 2550, absorption wavelength: 455 nm, solvent: ethanol). The items "zenoxitantin", "lutein", "α-carotene", "β-carotene", and "lycopene" in Fig. 1 are respectively based on values obtained by actually measuring, by high-performance liquid chromatography, the contents of zeaxanthin, lutein, α-carotene, β-carotene, and lycopene in each of the samples.

As shown in Fig. 1, CCD4-st1-1 and CCD4-st1-2 each contained 1.4 times to 2.1 times as much lutein, 1.4 times to 2.7 times as much β-carotene, and 2.5 times to 4.0 times as much zeaxanthin (each of which is a major carotenoid) as a corresponding one of the homozygous wild-type tobacco plants. Furthermore, CCD4-st1-1 and CCD4-st1-2 each exhibited 1.7 times to 2.5 times as high total carotenoid content as a corresponding one of the homozygous wild-type tobacco plants. No α-carotene or no lycopene was detected.

### (3b) Major carotenoid contents of CCD4 mutant of Nicotiana sylvestris grown on field

Seedlings for transplantation of the 08N-465 line obtained in Example 2 and a wild-type line (WT) thereof were grown in a greenhouse. After transplantation onto a field, management of growth and harvest was carried out under general conditions of growth of a tobacco leaf. Approximately one month after topping, two to three lower leaves harvested from each of approximately fifteen individuals in each of the lines were freeze-dried and then ground to obtain a sample. An analysis of the major carotenoid contents of each of samples was requested to Japan Food Research Laboratories. The results received from Japan Food Research Laboratories are summarized in Fig. 2.

As shown in Fig. 2, 08N-465 contained 1.4 time as much lutein, 1.3 times as much β-carotene, and 3.3 times as much zeaxanthin as WT. Furthermore, 08N-465 exhibited 1.7 times to 1.8 times as high total carotenoid content as WT. No α-carotene or no lycopene was detected.

### (3c) Carotenoid contents of cured leaves of double mutants grown on field

Seedlings for transplantation of two lines of double mutants (CCD4-st1-1 and CCD4-st1-2), two lines of homozygous wild-type tobacco plants (WT1 and WT2), which double mutants and homozygous wild-type tobacco plants had been obtained in Example 2, and the original variety (Tsukuba 1) were grown in a greenhouse. After transplantation onto a field, management was carried out under general conditions of growth of a tobacco leaf. Five cutter leaves were harvested from each of individuals on day 25 after topping, and subjected to an ordinary flue-curing process with use of a hot air dryer. Mid-rib parts of resulting cured leaves were removed, and lamina parts of the resulting cured leaves were ground to obtain a ground sample. In a 14-mL screw cap glass centrifuge tube, 0.1 g of the ground sample, 5.0 mL of an extraction solution (acetone : ethanol = 1:1 with addition of 2,6-di-tert-butylhydroxybenzene (0.1%), which was an antioxidant), and 5 µg of trans-retinol, which was an internal standard material, were introduced. After the centrifuge tube was capped with a screw cap, shake extraction (250 rpm, 25°C, 1 hour) was carried out with use of a medium constant temperature incubator shaker Bioshaker (registered trademark) BR-43FH MR (TAITEC Corp.). The extraction solution was filtered through a PTFE filter (pore diameter: 0.45 µm), and a filtrate was analyzed with use of a liquid chromatograph-tandem mass spectrometer (LC-MS/MS) equipped with a photodiode array detector (PDA). Devices, an instrument, and conditions employed in the analysis are as follows.

### (Chromatograph)

· High-performance liquid chromatograph: 1260 infinity (Agilent Technologies Japan, Ltd., Tokyo)
· PDA detector: 1260 infinity Diode Array Detector (Agilent Technologies Japan, Ltd., Tokyo)
· Column: ACQUITY UPLC (registered trademark) BEH 130 Å C18 (inner diameter: 2.1 mm, length: 100 mm, particle diameter: 1.7 µm; Nihon Waters K.K., Tokyo)
· Column temperature: 35°C
· Eluent A: acetonitrile : methanol = 7:3 (v/v)
· Eluent B: ultrapure water
· Elution condition:
   (1) 0 minute to 4 minutes: eluent A and eluent B (mixed solution at 85:15, for 4 minutes)
   (2) 4 minutes to 6 minutes: eluent A and eluent B (mixed solution at 85:15 to 100:0 with a linear concentration gradient, 2 minutes)
   (3) 6 minutes to 28.2 minutes: eluent A only (for 22.2 minutes)
· Flow rate: 0.2 mL/min
· Injection amount: 2.0 µL
· PDA detection wavelength: 325 nm (trans-retinol), 450 nm (carotenoids)

### (Mass Spectrometry)

· Mass spectrometer: 6470 Triple Quad LC/MS (Agilent Technologies Japan, Ltd., Tokyo)
· Ion source parameters
Ionization method: electro-spray ionization (ESI)
Nebulizer gas: nitrogen
Nebulizer gas temperature: 300°C
Nebulizer gas flow rate: 10 L/min
Nebulizer pressure: 35 psi
Sheath gas: 400°C
Sheath gas flow rate: 12 L/min
Capillary voltage: 3500 V
· Mass spectrometer parameters
Ion polarity: positive
Collision gas: nitrogen
Measurement mode: Selected reaction monitoring (SRM)

A combination (SRM transition) of a precursor ion and a product ion of each of carotenoids to be analyzed by SRM was set as shown in Table 3. In Table 3, a residence time of each of compounds is 42 msec, and a cell acceleration voltage is 3 V.

**[Table 3]**

| Compound name | Retention time (minutes) | Molecular weight | Precursor ion (Q1) | Product ion (Q3) | Fragmentor voltage (V) | Collision energy (eV) |
|---|---|---|---|---|---|---|
| Neoxanthin | 4.4 | 600.4 | 600.4 | 582.4 | 130 | 11 |
| Violaxanthin | 4.8 | 600.4 | 601.4 | 221.2 | 100 | 20 |
| trans-retinol (internal standard material) | 5.2 | 286.2 | 269.4 | 95.1 | 50 | 5 |
| Antheraxanthin | 7.0 | 584.4 | 584.4 | 492.4 | 160 | 10 |
| Zeaxanthin | 8.9 | 568.4 | 568.4 | 476.4 | 150 | 12 |
| Lutein | 9.0 | 568.4 | 568.4 | 476.4 | 170 | 15 |
| α-cryptoxanthin | 13.5 | 552.4 | 552.4 | 460.4 | 150 | 10 |
| β-cryptoxanthin | 13.8 | 552.4 | 552.4 | 460.3 | 80 | 15 |
| Lycopene | 16.6 | 536.4 | 536.4 | 444.5 | 105 | 11 |
| α-carotene | 21.7 | 536.4 | 536.4 | 444.2 | 100 | 20 |
| β-carotene | 22.6 | 536.4 | 536.4 | 444.9 | 170 | 15 |

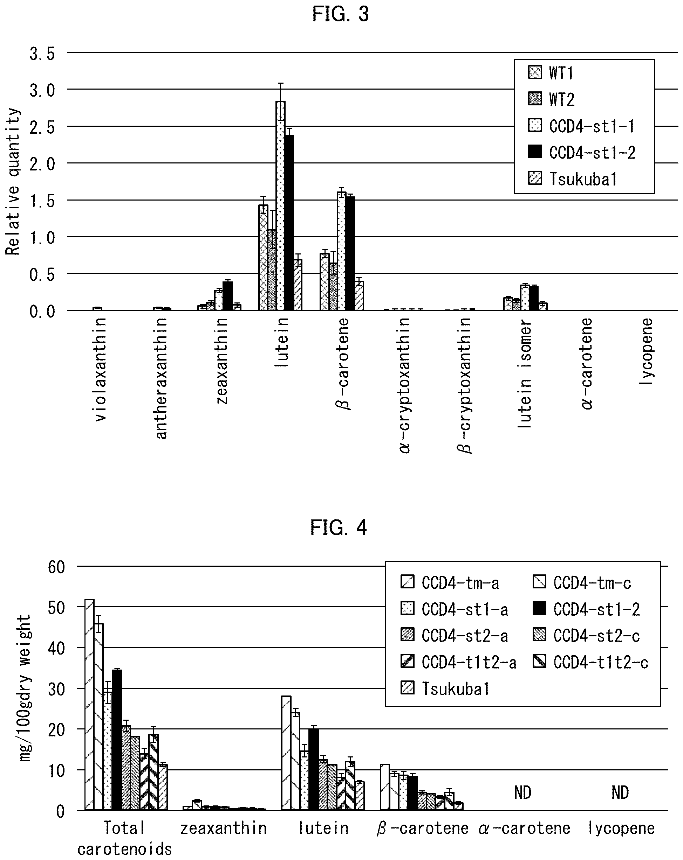

Each of carotenoid components was identified by comparing the retention time of the peak, which had been detected by a PDA and SRM analysis, with the retention time of the peak of a standard material. The content of each of the carotenoids was calculated while a value of the area value of an absorption spectrum of the each of the carotenoids, which absorption spectrum was detected at an optical wavelength of 450 nm, relative to the area value of an absorption spectrum of trans-retinol (internal standard material), which absorption spectrum was detected at an optical wavelength of 325 nm, was used as a semi-quantitative value. The carotenoid contents of each of the lines are summarized in Fig. 3. In Fig. 3, one carotenoid content of one line is shown as the average of values indicative of the carotenoid contents of three samples obtained from three zones on the field. As shown in Fig. 3, CCD4-st1-1 and CCD4-st1-2 contained 2.0 times or more as much lutein, 2.1 times or more as much β-carotene, and 3.8 times or more as much zeaxanthin as three control lines (WT1, WT2, and Tsukuba 1).

### (3d) Apocarotenoid contents of cured leaves of double mutants grown on field

In the following manner, 17 kinds of apocarotenoid contents of each of the ground samples described in (3c) were examined. In a 14-mL screw cap glass centrifuge tube, 0.1 g of the ground sample and 6.0 mL of an extraction solution (methanol with addition of 1,3-dimethoxybenzene (5.4 µg/mL) which was an internal standard material) were introduced. After the centrifuge tube was capped with a screw cap, shake extraction (250 rpm, 70°C, 1 hour) was carried out with use of a medium constant temperature incubator shaker Bioshaker (registered trademark) BR-43FH MR (TAITEC Corp.). The extraction solution was filtered through a PTFE filter having a pore diameter of 0.45 µm, and a filtrate was analyzed with use of a gas chromatogram-mass spectrometer (GC/MS). A device, instruments, and conditions employed in the analysis are as follows.
· Gas chromatogram-mass spectrometer: GC/MS 5977A MSD (Agilent Technologies Japan, Ltd., Tokyo)
· Column: HP-1ms (inner diameter: 0.25 mm, length: 30 m; film thickness: 0.25 µm; Agilent Technologies Japan, Ltd., Tokyo)
· Insert liner: splitless liner with deactivated glass wool (Agilent Technologies Japan, Ltd., Tokyo)
· Helium flow rate: 1.0 mL/min
· Injection amount: 1.0 µL
· Injection mode: splitless
· Insert temperature: 280°C
· AUX temperature: 280°C
· Column temperature rise conditions:
   (1) keep temperature at 50°C for 1 min
   (2) raise temperature from 50°C to 100°C at 10°C/min
   (3) raise temperature from 100°C to 200°C at 2°C/min
   (4) raise temperature from 200°C to 300°C at 20°C/min
   (5) keep temperature at 300°C for 19 min
· Analysis mode: TIC/SIM mode

TIC was analyzed in an m/z range of 30 to 500. A combination (SIM parameter) of a semi-quantitative ion and a qualitative ion(s) of each of the apocarotenoids to be analyzed by SIM was set as shown in Table 4. In Table 4, the compounds for which parenthesized numbers are shown at the ends represent structural isomers.

**[Table 4]**

| Compound name | Retention time (minutes) | Semi-quantitative ion | Qualitative ion | |
|---|---|---|---|---|
| 1,3-dimethoxybenzene (internal standard material) | 9.8 | 138 | 109 | 95 |
| β-cyclocitral | 11.6 | 137 | 152 | 123 |
| β-damasce none | 18.1 | 69 | 121 | 190 |
| β-damascone | 19.5 | 177 | 192 | 123 |
| Oxoedulan (2) | 21.2 | 193 | 109 | 208 |
| Oxoedulan (1) | 22.4 | 193 | 190 | 208 |
| 8,9-dehydrotheaspirone | 22.7 | 108 | 206 | 150 |
| β-ionone | 22.9 | 177 | 192 | |
| Dihydroactinidiolide | 23.6 | 111 | 137 | 180 |
| Megastigmatrienone (1) | 26.3 | 190 | 175 | 147 |
| Megastigmatrienone (2) | 27.3 | 190 | 175 | 147 |
| 3-hydroxy-β-damascone | 28.8 | 175 | 193 | 208 |
| Megastigmatrienone (4) | 28.8 | 190 | 175 | 147 |
| Megastigmatrienone (3) | 29.5 | 190 | 175 | 147 |
| 3-hydroxy-7,8-dehydro-β-ionol | 29.9 | 193 | 208 | 175 |
| 3-oxo-a-ionol | 30.3 | 108 | 152 | |
| 3-hydroxy-5,6-epoxy-β-ionol | 31.6 | 125 | 208 | 43 |
| Blumenol C | 33.0 | 135 | 150 | 210 |
| 4-(3-hydroxybutylidene)-3,5,5-trimethyl-2-cyclohexan-1-one | 33.5 | 149 | 164 | |
| Blumenol A | 37.1 | 124 | 135 | 168 |

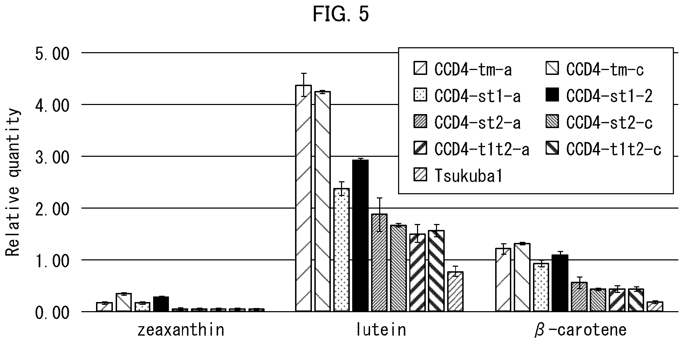

Each of apocarotenoid components was identified by checking, with information in literatures and information in database, a full mass spectrum that had been obtained in a TIC mode and that was at a retention time at which the semi-quantitative ion and the qualitative ions were detected in an SIM mode. The content of each of the apocarotenoids was calculated while a value of the area value of a chromatogram of the semi-quantitative ion of the each of the apocarotenoids relative to the area value of a chromatogram of the semi-quantitative ion of the 1,3-dimethoxybenzene, which was an internal standard material, was used as a semi-quantitative value. The carotenoid contents of each of the lines are summarized in Fig. 5. In Fig. 5, one carotenoid content of one line is shown as the average of values indicative of the carotenoid contents of three samples obtained from three zones on the field, and "ND" indicates "Not detected". As shown in Table 5, CCD4-st1-1 and CCD4-st1-2 contained β-ionone and dihydroactinidiolide (compounds in lines 1 and 2) which were increased to approximately 2 times to 3 times, apocarotenoids (compounds in lines 3 to 8) which were decreased to approximately 1/2 to 1/3, and apocarotenoids (compounds in lines 9 to 17) which did not show clear changes, as compared with three control lines (WT1, WT2, and Tsukuba 1). The apocarotenoids which were increased (β-ionone and dihydroactinidiolide) and the apocarotenoids which were decreased (compounds in lines 3 to 8) are each known as an aromatic apocarotenoid.

**[Table 5]**

| | WT1 | WT2 | CCD4-st1-1 | CCD4-st1-2 | Tsukuba1 |
|---|---|---|---|---|---|
| dihydroactinidiolide | 0.0068 | 0.0044 | 0.0124 | 0.0125 | 0.0036 |
| *β*-ionone | 0.0029 | 0.0027 | 0.0060 | 0.0053 | 0.0019 |
| *β*-damascenone | 0.0069 | 0.0071 | 0.0037 | 0.0036 | 0.0062 |
| Megastigmatrienone(1) | 0.0234 | 0.0257 | 0.0149 | 0.0133 | 0.0261 |
| Megastigmatrienone (2) | 0.0637 | 0.0710 | 0.0372 | 0. 0322 | 0.0721 |
| Megastigmatrienone (3) | 0.0950 | 0.1047 | 0.0554 | 0.0498 | 0.1088 |
| Megastigmatrienone (4) | 0.0554 | 0.0570 | 0.0190 | 0.0175 | 0.0379 |
| 3-Hydroxy-b-damascone | 0.0473 | 0.0496 | 0.0232 | 0.0239 | 0.0533 |
| 3-hydroxy-5,6-epoxy-b-ionol | 0.0303 | 0.0170 | 0.0281 | 0.0085 | 0.0101 |
| 4-(3-hydroxybuthylidene)-3,5,5-trimethyl | 0.0067 | 0.0040 | 0.0063 | 0.0018 | 0.0024 |
| oxo-eduran-I | 0.0003 | ND | 0.0003 | ND | ND |
| *β*-cyclocitral | 0.0007 | 0.0008 | 0.0011 | 0.0008 | 0.0005 |
| 3-hydroxy-7,8-dehydro-b-ionol | 0.0040 | 0.0019 | 0. 0031 | 0.0015 | 0.0012 |
| Blumenol_A | 0.0437 | 0.0272 | 0.0442 | 0.0165 | 0.0159 |
| Blumenol_C | 0.0081 | 0.0053 | 0.0106 | 0.0032 | 0.0029 |
| 3-oxo-a-ionol | 0.1260 | 0.0650 | 0.1571 | 0. 0472 | 0.0360 |
| *β*-damascone | 0.0032 | 0.0041 | 0. 0031 | 0. 0029 | 0.0031 |

| | | | | | |
|---|---|---|---|---|---|
| ND:Not detected | | | | | |

### [4. Sensory evaluation test of cured leaves]

### (4a) Sensory evaluation test for cigarettes

Cigarettes were prepared with use of a cigarette making machine and cured leaves which had been obtained from leaves harvested from two lines of double mutants (CCD4-st1-1 and CCD4-st1-2), three lines of single mutants (CCD4-s-1, CCD4-t1-1, and CCD4-t1-2), and three lines of control plants (WT1, WT2, and Tsukuba 1) grown on a field. The aroma and taste of each of these cigarettes was scored on the basis of sensory evaluation performed by 10 trained panelists. The panelists were caused to answer sensibilities which the panelists felt as a result of smoking the cigarettes prepared from the double mutants and the single mutants, with respect to sensibilities which the panelists felt as a result of smoking the cigarettes prepared from the control plants. The kinds of answers were "good", "somewhat good", "no difference", "somewhat bad", and "bad" (as compared with the controls). "Good" was scored 2 points, "somewhat good" was scored 1 point, and "no difference", "somewhat bad", and "bad" were scored 0 point. In this manner, the aroma and taste of each of the cigarettes was scored. Table 6 shows the results of totaling up the scores. As shown in Table 6, the aroma and taste of CCD4-st1-1 and CCD4-st1-2 was evaluated as "good" or "somewhat good" by 7 out of 10 panelists. The total score of each of CCD4-st1-1 and CCD4-st1-2 was higher than that of each of CCD4-s-1, CCD4-t1-1, and CCD4-t1-2. These results indicate that the cured leaves which had been obtained from the leaves harvested from the double mutants provided a pleasant stimulus to human's sense organs, as compared with the cured leaves which had been obtained from the leaves harvested from the single mutants and the control plants.

**[Table 6]**

| | Good | Somewhat good | No difference | Somewhat bad | Bad | Total points |
|---|---|---|---|---|---|---|
| | 2 points | 1 point | 0 point | 0 point | 0 point | |
| CCD4-st1-1 | 4 | 3 | 2 | 1 | 0 | 11 |
| CCD4-st1-2 | 2 | 5 | 1 | 1 | 1 | 9 |
| CCD4-s-1 | 1 | 4 | 4 | 1 | 0 | 6 |
| CCD4-t1-1 | 1 | 2 | 1 | 4 | 2 | 4 |
| CCD4-t1-2 | 0 | 4 | 3 | 2 | 1 | 4 |

### (4b) Sensory evaluation test for finely-ground samples

The cured leaves obtained in (4a) were ground and further finely ground. The resulting finely ground cured leaves were then dispersed in polyethylene glycol (3 times the mass of the finely ground cured leaves) to obtain finely ground samples. Three experienced panelists (more trained and having more experience than the above panelists) carried out sensory evaluation (comparison of test objects with a control). The control was a non-aromatized product of MEVIUS (trademark), and the test objects were each a product obtained by spreading a corresponding one of the finely ground samples on the non-aromatized product of MEVIUS (trademark). The panelists were caused to smoke the control and the test objects and answer in regard to differences in aroma and taste between the test objects and the control. The experienced panelist's answer that "an improvement in floral aroma and taste was perceived" was scored 1 point, and the experienced panelist's answer that "an improvement in floral aroma and taste was not perceived" was scored 0 point. The results of scoring the test objects are summarized in Table 7. As shown in Table 7, all of the panelists perceived an improvement in floral aroma and taste with respect to CCD4-st1-1, and two out of three panelists perceived an improvement of floral aroma and taste with respect to CCD4-st1-2. The above results indicate that the cured leaves which had been obtained from the leaves harvested from the double mutants can add good aroma and taste to conventional tobacco products.

**[Table 7]**

| | WT1 | WT2 | CCD4-st1-1 | CCD4-st1-2 | Tsukuba 1 |
|---|---|---|---|---|---|
| Total points | 0 | 0 | 3 | 2 | 0 |

### [5. Further preparation of single mutant (CCD4-t2), double mutants (CCD4-st2 and CCD4-tlt2), and triple mutant (CCD4-tm) each having mutation(s) in CCD4 gene(s)]

The following three lines each having a mutation in a CCD4-T2 gene were selected by analyzing the DNA sequences of an EMS mutant population (M2 generation, approximately 2000 lines) of tobacco (Tsukuba 1) and determining the sequences by amplicon sequencing.
Line name: 1795 line (hereinafter referred to as "CCD4-t2-1")
Line name: 0799 line (hereinafter referred to as "CCD4-t2-2")
Line name: 1352 line (hereinafter referred to as "CCD4-t2-3")

CCD4-t2-1 had a nonsense mutation (nucleotide substitution in which a codon encoding the 190th arginine (R) changed to a stop codon) in the CCD4-T2 gene. CCD4-t2-2 had a splicing mutation (a base G at the 5' end of the first intron was replaced with A) in the CCD4-T2 gene. CCD4-t2-3 had a splicing mutation (a base G at the 3' end of the first intron was replaced with A) in the CCD4-T2 gene. The above selected three lines were grown. As a result, yellowing was not observed in mature (aged) lower leaves of any of the lines. CCD4-t2-3 was used to prepare a triple mutant having mutations in CCD4 genes.

Next, a double mutant and a triple mutant each homozygously having intended mutations in CCD4 genes were prepared through two-step crossing and selfing.
First Step: preparation of CCD4-st1-2 (having homozygous mutations in a CCD4-S gene and a CCD4-T1 gene, respectively) by crossing CCD4-s-1 with CCD4-t1-2 (Example 2)
Second step: preparation of a preliminary mutant line (having heterozygous mutations in a CCD4-S gene, a CCD4-T1 gene, and a CCD4-T2 gene, respectively) by crossing CCD4-st1-2 with CCD4-t2-3. Selfing: preparation of CCD4-058 by selfing of the above mutant line (observed genotype will be described later)

Mutations in the CCD4 genes present in genomes of each of 360 individual seedlings developed from seeds of the CCD4-058 line were identified by sequence determination. By this identification, it was confirmed that double mutants (CCD4-st1, CCD4-st2, and CCD4-tlt2) each having homozygous mutations in two CCD4 genes and a triple mutant (CCD4-tm) having homozygous mutations in three CCD4 genes were present in 360 individuals of progeny of the CCD4-058 line (seeds of selfed progeny were obtained from these mutants). Note that a region of the CCD4-T2 gene which region contains the mutation was amplified with use of the following primers.
Tobacco CCD4-T2 forward primer: CAAACTCCAGCGGAGATA (SEQ ID NO: 22)
Tobacco CCD4-T2 reverse primer: GGCACGCCACTATGCAAT (SEQ ID NO: 23)

The resulting double mutants (CCD4-st1, CCD4-st2, and CCD4-tlt2) and the triple mutant (CCD4-tm) were developed in a greenhouse, and the colors of mature (aged) lower leaves thereof were visually observed. In regard to CCD4-st2, CCD4-tlt2, and CCD4-tm, the colors were orange to yellow, but the intensity of the colors differed between the lines depending on their CCD4 genotypes. The colors became more intense in order of (i) CCD4-tm, (ii) CCD4-st1, and (iii) CCD4-st2 and CCD4-tlt2 (comparable).

### [6. Analysis of colors of mature (aged) leaves of CCD4 double mutants and triple mutant]

### (6a) Analysis of colors of fresh leaves

Six lines (three genotypes × two lines) of double mutants (CCD4-st1-a, CCD4-st1-2 (see Examples 3 and 4), CCD4-st2-a, CCD4-st2-c, CCD4-tlt2-a, and CCD4-tlt2-c), two lines of triple mutants (CCD4-tm-a and CCD4-tm-c), and one line of a control plant (Tsukuba 1) were grown in a greenhouse at 23°C to 25°C. On day 94 after seeding (day 8 after topping), fresh mature (aged) lower leaves (one leaf from each of three individuals per line) were collected, and the colors of the fresh mature (aged) lower leaves were measured with use of a colorimeter CR-20 (Konica Minolta Japan, Inc., Tokyo). The L*a*b* system employed by the Commission internationale de l'eclairage and JIS was used to express a color by values. The colorimeter CR-20 outputs values which constitute the L*a*b* system (employed by the Commission internationale de l'eclairage and JIS). The results of analyzing the colors are shown in Table 8.

**[Table 8]**

| | L∗ | a∗ | b∗ |
|---|---|---|---|
| CCD4-tm-a | 76. 4 | 19.0 | 64.4 |
| CCD4-tm-c | 76.2 | 19.5 | 63.6 |
| CCD4-st1-a | 78. 7 | 14.0 | 59.5 |
| CCD4-st1-2 | 78.4 | 14.8 | 60.6 |
| CCD4-st2-a | 82.0 | 6.5 | 43.4 |
| CCD4-st2-c | 83.3 | 4.2 | 37. 6 |
| CCD4-t1t2-a | 82. 3 | 4.8 | 37.6 |
| CCD4-t1t2-c | 84.6 | 2.7 | 33.7 |
| Tsukuba1 | 84. 2 | 2.3 | 33. 6 |

Each of values shown in Table 8 is the average of measurements outputted with respect to 18 measurement points per line (3 fresh leaves). The breakdown of the measurement points is as follows: 6 points on a surface of one fresh leaf (an upper part, a middle part, and a lower part of each of right and left halves into which a leaf is divided with the main vein as the axis of symmetry) × 3. A negative value in a* represents the intensity of green, and a positive value in a* represents the intensity of red/magenta. A negative value in b* represents the intensity of blue, and a positive value in b* represents the intensity of yellow. A value in L* represents (the height of) brightness.

As shown in Table 8, in the triple mutants, more intense yellowing (development of orange to yellow) than in the double mutants was observed. Among the double mutants, the line (CCD4-st1) having mutations in a CCD4-S gene and a CCD4-T1 gene showed the most intense yellowing. In the other lines (CCD4-st2 and CCD4-tlt2), slightly weak yellowing (more intense than in Tsukuba 1, which was a control plant) was observed. These results were consistent with the visual observation described above.

### (6b) Analysis of colors of cured leaves

Seedlings for transplantation of each of the lines used in (6a) were grown in a greenhouse. General conditions of growth of a tobacco leaf were employed for management of transplantation onto a field, growth, and harvest. Five cutter leaves were harvested from each of individuals on day 16 after topping, and subjected to an ordinary flue-curing process with use of a hot air dryer. Tendencies similar to those observed in the fresh leaves in Example 5 were visually observed also in the cured leaves.

The colors of the cured leaves were analyzed in the same manner as in (6a), except for the locations and the number of measurement points. The number of measurement points per line was 24 (4 cured leaves (leaves were collected from respective four test plots) per line were analyzed). The results of analyzing the colors are shown in Table 9. As is clear from Tables 8 and 9, the fresh leaves and the cured leaves (obtained from the double mutants and the triple mutant) showed the same tendencies as to yellowing.

**[Table 9]**

| | L∗ | a∗ | b∗ |
|---|---|---|---|
| CCD4-tm-a | 68.1 | 18.0 | 50.6 |
| CCD4-tm-c | 65.8 | 20.3 | 53.4 |
| CCD4-st1-a | 69.0 | 16.7 | 50.1 |
| CCD4-stl-2 | 68.8 | 17.2 | 51.1 |
| CCD4-st2-a | 68.2 | 14.5 | 47.8 |
| CCD4-st2-c | 68.2 | 15.5 | 47.4 |
| CCD4-t1t2-a | 69.2 | 14.8 | 48.2 |
| CCD4-t1t2-c | 69.8 | 13.3 | 44.4 |
| Tsukuba1 | 70.5 | 11.1 | 38.6 |

### [7. Quantitative analyses of carotenoids and apocarotenoids in CCD4 double mutants and triple mutants]

### (7a) Major carotenoid contents of double mutants and triple mutants grown in greenhouse

The following nine lines (one from each of lines shown in parentheses) were grown in a greenhouse at 23°C to 25°C:
double mutants each having mutations in st1 (CCD4-st1-a and CCD4-st1-2);
double mutants each having mutations in st2 (CCD4-st2-a and CCD4-st2-c);
double mutants each having mutations in tlt2 (CCD4-tlt2-a and CCD4-tlt2-c);
triple mutants (CCD4-tm-a and CCD4-tm-c); and
a control (Tsukuba 1).
Four to five lower leaves harvested on day 8 to 10 from topping were frozen with use of liquid nitrogen, and stored at -80°C. The lower leaves were further treated (freeze-dried and ground), and then subjected to a major carotenoid analysis. The analysis was requested to Japan Food Research Laboratories. The results of the analysis are shown in Fig. 4. Each bar in Fig. 4 represents the average content of a component (in three individuals) shown at the bottom. Each bar for CCD4-tm-a represents the content of the component in one individual, and each bar for CCD4-st2-c represents the average content of the component in two individuals.

As shown in Fig. 4, the double mutants and the triple mutants each showed higher carotenoid contents than Tsukuba 1. The degrees of elevation of the carotenoid contents differed from each other depending on genotypes (presence or absence of mutations in CCD4-S, -T1, and -T2 genes). Tendencies for the degrees to differ from each other depending on the genotypes were similar among the components (total carotenoid, lutein, zeaxanthin, and β-carotene). As one example, the content of lutein became higher in order of CCD4-tm (3.4 times to 4.0 times), CCD4-st1 (2.1 times to 2.8 times), CCD4-st2 (1.6 times to 1.8 times), and CCD4-tlt2 (1.2 times to 1.7 times). The magnifications in parentheses are those when the component content of Tsukuba 1 is regarded as 1.

### (7b) Carotenoid contents of cured leaves of double mutants and triple mutants grown on field

The same nine lines as in (7a) were grown in a greenhouse. General conditions of growth of a tobacco leaf were employed for management of transplantation onto a field, growth, and harvest. Five cutter leaves were harvested from each of individuals on day 16 after topping, and subjected to an ordinary flue-curing process with use of a hot air dryer. Mid-ribs of resulting cured leaves were removed, and remaining lamina parts of the resulting cured leaves were ground to obtain a sample to be subjected to a semi-quantitative analysis. The semi-quantitative analysis of carotenoids was carried out as described in (3c). The results are shown in Fig. 5. Each bar in Fig. 5 represents the average content of a component (in three individuals) shown at the bottom.

As shown in Fig. 5, the results of the semi-quantitative analysis were consistent with the results shown in Fig. 4. As one example, the content of lutein became higher in order of CCD4-tm (5.5 times to 5.7 times), CCD4-st1 (3.1 times to 3.8 times), CCD4-st2 (2.2 times to 2.4 times), and CCD4-tlt2 (2.0 times). The magnifications in parentheses are those when the component content of Tsukuba 1 is regarded as 1.

(7c) The apocarotenoid contents of cured leaves of each of double mutants and triple mutants grown on field and 17 kinds of apocarotenoids in each of cured leaf samples were semiquantitatively analyzed. The cured leaf sample was prepared as described in (7b), and the analysis was carried out as described in (3c). The results of the semi-quantitative analysis are shown in Table 10. Each values shown in Table 10 is the average for one line (three individuals).

**[Table 10]**

| | CCD4-tm-a | CCD4-tm-c | CCD4-st1-a | CCD4-st1-2 | CCD4-st2-a | CCD4-st2-c | CCD4-t1t2-a | CCD4-t1t2-c | Tsukubal |
|---|---|---|---|---|---|---|---|---|---|
| dihydroactinidiolide | 0.1018 | 0.0905 | 0.0476 | 0.0506 | 0.0302 | 0.0258 | 0.0269 | 0.0366 | 0.0189 |
| β-ionone | 0.0343 | 0.0334 | 0.0220 | 0.0277 | 0.0117 | 0.0117 | 0.0119 | 0.0184 | 0.0096 |
| β-damascenone | 0.0172 | 0.0133 | 0.0171 | 0.0183 | 0.0286 | 0.0297 | 0.0301 | 0.0264 | 0.0442 |
| Megastigmatrienone(1) | 0.0280 | 0.0339 | 0.1325 | 0.1473 | 0.1608 | 0.1701 | 0.1715 | 0.1803 | 0.2817 |
| Megastigmatrienone(2) | 0.0512 | 0.0637 | 0.3502 | 0.4151 | 0.4505 | 0.4790 | 0.4830 | 0.5203 | 0.8068 |
| Megastigmatrienone(3) | 0.0831 | 0.0965 | 0.5042 | 0.5683 | 0.6209 | 0.6541 | 0.6754 | 0.7247 | 1.0952 |
| Megastigmatrienone(4) | 0.0327 | 0.0386 | 0.1785 | 0.1962 | 0.2234 | 0.2341 | 0.2382 | 0.2524 | 0.3818 |
| 3-Hydroxy-b-damascone | 0.0726 | 0.0730 | 0.1863 | 0.1878 | 0.2645 | 0.2855 | 0.2756 | 0.2732 | 0.4185 |
| 3-hydroxy-5,6-epoxy-b-ionol | 0.0582 | 0.0351 | 0.0458 | 0.0479 | 0.0595 | 0.0640 | 0.0684 | 0.0730 | 0.1616 |
| 4-(3-hydroxybuthylidene)-3,5,5-trimethyl | 0.0085 | 0.0057 | 0.0152 | 0.0131 | 0.0095 | 0.0105 | 0.0114 | 0.0125 | 0.0210 |
| oxo-eduran-I | ND | ND | ND | ND | ND | ND | ND | ND | ND |
| 13-cyclocitral | 0.0056 | 0.0058 | 0.0061 | 0.0071 | 0.0058 | 0.0049 | 0.0042 | 0.0061 | 0.0035 |
| 3-hydroxy-7,8-dehydro-b-ionol | 0.0064 | 0.0025 | 0.0023 | 0.0024 | 0.0037 | 0.0046 | 0.0050 | 0.0035 | 0.0063 |
| Blumenol_A | 0.0857 | 0.0533 | 0.0805 | 0.0871 | 0.1166 | 0.1000 | 0.0952 | 0.1182 | 0.1563 |
| Blumenal_C | 0.0044 | 0.0024 | 0.0059 | 0.0112 | 0.0059 | 0.0071 | 0.0099 | 0.0109 | 0.0162 |
| 3-oxo-a-ionol | 0.1383 | 0.0735 | 0.2220 | 0.2099 | 0.1418 | 0.1615 | 0.1938 | 0.2094 | 0.4194 |
| β-damascone | 0.0007 | 0.0009 | 0.0090 | 0.0124 | 0.0103 | 0.0093 | 0.0088 | 0.0128 | 0.0131 |

| | | | | | | | | | |
|---|---|---|---|---|---|---|---|---|---|
| ND:Not detected | | | | | | | | | |

As shown in Table 10, each of the triple mutants produced the most pronounced change in the apocarotenoid contents, with respect to Tsukuba 1. The mutants shared increases in β-ionone and dihydroactinidiolide as in Example 3. Specifically, the content of β-ionone (magnifications in parentheses are based on Tsukuba 1 serving as 1) was 3.5 times to 3.6 times in the case of CCD4-tm, 2.3 times to 2.9 times in the case of CCD4-st1, 1.2 times in the case of CCD4-st2, and 1.2 times to 1.9 times in the case of CCD4-tlt2. The content of dihydroactinidiolide was 4.8 times to 5.4 times in the case of CCD4-tm, 2.5 times to 2.7 times in the case of CCD4-st1, 1.4 times to 1.6 times in the case of CCD4-st2, and 1.4 times to 1.9 times in the case of CCD4-tlt2. On the other hand, many of the apocarotenoids in the cured leaves of each of the triple mutants were each decreased to approximately 1/3 to 1/10 of a corresponding one of the apocarotenoids in Tsukuba 1.

### Industrial Applicability

The present invention can be used to improve the quality of a tobacco product.

## Claims

1. A tobacco plant in which a mutation is introduced in a genome, the mutation causing suppression of a function of at least any one of:
an endogenous gene which contains, as a coding region, a polynucleotide that encodes a polypeptide having a sequence identity of 80% or higher with an amino acid sequence represented by SEQ ID NO: 1;
an endogenous gene which contains, as a coding region, a polynucleotide that encodes a polypeptide having a sequence identity of 80% or higher with an amino acid sequence represented by SEQ ID NO: 2; and
an endogenous gene which contains, as a coding region, a polynucleotide that encodes a polypeptide having a sequence identity of 80% or higher with an amino acid sequence represented by SEQ ID NO: 3.

2. The tobacco plant as set forth in claim 1, wherein the suppression of the function is a decrease in an amount of mRNA which has been transcribed from the at least any one of the endogenous genes, as compared with a wild-type plant.

3. The tobacco plant as set forth in claim 1 or 2, wherein the suppression of the function is promotion of degradation of mRNA which has been transcribed from the at least any one of the endogenous genes.

4. The tobacco plant as set forth in any one of claims 1 through 3, wherein the mutation is introduced in the at least any one of the endogenous genes.

5. The tobacco plant as set forth in claim 4, wherein the mutation is introduced by mutagen treatment, genome editing, or gene knockout.

6. The tobacco plant as set forth in claim 5, wherein the mutation is introduced by the mutagen treatment.

7. The tobacco plant as set forth in claim 3, wherein the mutation is insertion, in an outside of a region in which the at least any one of the endogenous genes is present, of a polynucleotide which expresses a factor that promotes the degradation of the mRNA.

8. The tobacco plant as set forth in claim 7, wherein the factor is an antisense RNA molecule, an RNAi molecule, or a co-suppression molecule.

9. The tobacco plant as set forth in any one of claims 1 through 8, wherein the suppression of the function is a decrease in an amount of the polypeptide which has an original function, as compared with a wild-type plant.

10. The tobacco plant as set forth in claim 9, wherein the suppression of the function is a decrease in an amount of translation into the polypeptide which has the original function, as compared with a wild-type plant.

11. The tobacco plant as set forth in any one of claims 1 through 10, wherein the tobacco plant belongs to *Nicotiana tabacum, Nicotiana rustica,* or *Nicotiana sylvestris.*

12. A method of producing a tobacco plant, comprising the step of introducing a mutation into a genome of a tobacco plant, the mutation causing suppression of a function of at least any one of:
an endogenous gene which contains, as a coding region, a polynucleotide that encodes a polypeptide having a sequence identity of 80% or higher with an amino acid sequence represented by SEQ ID NO: 1;
an endogenous gene which contains, as a coding region, a polynucleotide that encodes a polypeptide having a sequence identity of 80% or higher with an amino acid sequence represented by SEQ ID NO: 2; and
an endogenous gene which contains, as a coding region, a polynucleotide that encodes a polypeptide having a sequence identity of 80% or higher with an amino acid sequence represented by SEQ ID NO: 3.

13. The method as set forth in claim 12, wherein the suppression of the function is a decrease in an amount of mRNA which has been transcribed from the at least any one of the endogenous genes, as compared with a wild-type plant.

14. The method as set forth in claim 12 or 13, wherein the suppression of the function is promotion of degradation of mRNA which has been transcribed from the at least any one of the endogenous genes.

15. The method as set forth in any one of claims 12 through 14, wherein the step of introducing the mutation includes introducing the mutation into the at least any one of the endogenous genes.

16. The method as set forth in claim 15, wherein the step of introducing the mutation is carried out by mutagen treatment, genome editing, or gene knockout.

17. The method as set forth in claim 16, wherein the mutation is introduced by the mutagen treatment.

18. The method as set forth in any one of claims 12 through 15, wherein the step of introducing the mutation includes inserting, into an outside of a region in which the at least any one of the endogenous genes is present, a polynucleotide which expresses a factor that promotes the degradation of the mRNA having been transcribed from the at least any one of the endogenous genes.

19. The method as set forth in claim 18, wherein the factor is an antisense RNA molecule, an RNAi molecule, or a co-suppression molecule.

20. The method as set forth in any one of claims 12 through 19, wherein the suppression of the function is a decrease in an amount of the polypeptide which has an original function, as compared with a wild-type plant.

21. The method as set forth in claim 20, wherein the suppression of the function is a decrease in an amount of translation into the polypeptide which has the original function, as compared with a wild-type plant.

22. Offspring or bred progeny, the offspring being of a tobacco plant recited in any one of claims 1 through 11 or a tobacco plant produced by a method recited in any one of claims 12 through 21, the bred progeny being obtained by crossing a tobacco plant recited in any one of claims 1 through 11 or a tobacco plant produced by a method recited in any one of claims 12 through 21 with another tobacco plant.

23. A tobacco leaf which is obtained from a tobacco plant,
in the tobacco leaf, a mutation being introduced in a genome, the mutation causing suppression of a function of at least any one of:
an endogenous gene which contains, as a coding region, a polynucleotide that encodes a polypeptide having a sequence identity of 80% or higher with an amino acid sequence represented by SEQ ID NO: 1;
an endogenous gene which contains, as a coding region, a polynucleotide that encodes a polypeptide having a sequence identity of 80% or higher with an amino acid sequence represented by SEQ ID NO: 2; and
an endogenous gene which contains, as a coding region, a polynucleotide that encodes a polypeptide having a sequence identity of 80% or higher with an amino acid sequence represented by SEQ ID NO: 3,
the tobacco leaf having a higher total carotenoid content than a tobacco leaf which is obtained from a wild-type tobacco plant.

24. A cured leaf which is obtained from a tobacco plant,
in the cured leaf, a mutation being introduced in a genome, the mutation causing suppression of a function of at least any one of:
an endogenous gene which contains, as a coding region, a polynucleotide that encodes a polypeptide having a sequence identity of 80% or higher with an amino acid sequence represented by SEQ ID NO: 1;
an endogenous gene which contains, as a coding region, a polynucleotide that encodes a polypeptide having a sequence identity of 80% or higher with an amino acid sequence represented by SEQ ID NO: 2; and
an endogenous gene which contains, as a coding region, a polynucleotide that encodes a polypeptide having a sequence identity of 80% or higher with an amino acid sequence represented by SEQ ID NO: 3,
the cured leaf having a higher total carotenoid content than a cured leaf which is obtained from a wild-type tobacco plant.

25. A tobacco product comprising a cured leaf recited in claim 24.
